Europäisches Patentamt

European Patent Office

Office européen des brevets

(11) Veröffentlichungsnummer: **0 151 937**
**B1**

(12) **EUROPÄISCHE PATENTSCHRIFT**

(45) Veröffentlichungstag der Patentschrift: **29.08.90**

(51) Int. Cl.⁵: **C 08 L 75/00**, C 08 J 9/00, C 08 K 3/04

(21) Anmeldenummer: **85100288.1**

(22) Anmeldetag: **14.01.85**

(54) **Verfahren zur Herstellung von schaumstoffhaltigen Polyurethan(harnstoff)-Massen, schaumstoffhaltige Polyurethan(harnstoff)-Massen und ihre Verwendung.**

(30) Priorität: **26.01.84 DE 3402696**

(43) Veröffentlichungstag der Anmeldung:
**21.08.85 Patentblatt 85/34**

(45) Bekanntmachung des Hinweises auf die Patenterteilung:
**29.08.90 Patentblatt 90/35**

(84) Benannte Vertragsstaaten:
**AT BE CH DE FR GB IT LI NL SE**

(56) Entgegenhaltungen:
**EP-A-0 031 425**
**DE-A-2 920 525**
**FR-A-2 482 127**
**US-A-3 805 532**
**US-A-4 082 703**

(73) Patentinhaber: **BAYER AG**
**D-5090 Leverkusen 1 Bayerwerk (DE)**

(72) Erfinder: **Reischl, Artur, Dr.**
**H.T.-von-Böttinger-Strasse 19**
**D-5090 Leverkusen 1 (DE)**

**Die Akte enthält technische Angaben, die nach dem Eingang der Anmeldung eingereicht wurden und die nicht in dieser Patentschrift enthalten sind.**

**Beschreibung**

Gegenstand der Erfindung ist ein Verfahren zur Herstellung von vorgefertigte Schaumstoffe, vorzugsweise in stückiger Form, und weitere Füllstoffe, insbesondere Baunkohlenstaub und/oder Torf, als Füllstoffe enthaltenden, kationisch modifizierten, stückigen oder flächigen, die Füllstoffe abriebfest gebunden enthaltenden Polyurethan(harnstoff)-Massen mit einem sehr hohen Wasseraufnahmefähigkeitswert (WAF).

Die Herstellung erfolgt durch Umsetzung von kationisch modifizierten NCO-Prepolymeren einer Funktionalität von mehr als 2,1, mit einer weit überstöchiometrischen Mengen an Wasser in Gegenwart von zerkleinerten, vorgefertigten Schaumstoffen, insbesondere PU-Schaumstoffen, und zusätzlichen Füllstoffen vorzugsweise zusätzlich Braunkohlenpulver und/oder Torf, gegebenenfalls in Gegenwart von weiteren, anorganischen oder organischen Füllstoffen und gegebenenfalls in Gegenwart von Biomassen (lebenden Zellen, lebenden Bakterien, Enzymen).

Die entsprechenden Polyurethan(harnstoff)-Massen binden mittels mindestens 5 Gew.-% an Polyurethan(harnstoff)-Matrix bis zu 95 Gew.-% an vorgefertigten Schaumstoffteilchen und weiteren Füllstoffen, vorzugsweise Braunkohle und/oder Torf, in der füllstoffhaltigen Polyurethan(harnstoff)-Masse (Feststoff) und sind von der Herstellung bereits gequollen bzw. stark wasserhaltig. Ihr Wasseraufnahmefähigkeitswert (WAF) bei Suspendierung in Wasser beträgt 30 bis 97 Gew.-% Wasser. Sie finden daher — in gegebenenfalls Biomassen eingebaut enthaltender Form-Verwendung als Träger in mikrobiellen Syntheseverfahren zur Herstellung komplizierter organischer Verbindungen, als spezielle Träger für das Wachstum von Pflanzen, als Filtermittel oder als Adsorbentien für nicht wasserlösliche Flüssigkeiten, z.B. (Roh)Oel oder Benzin.

Es wurden schon eine Reihe von Verfahren zur Imprägnierung von Schaumstoffen und Schaumstoffteilchen beschrieben, indem man die Schaumstoffe mit einer Reaktivkomponente, z.B. mit Polyisocyanaten, tränkt und nachträglich der Umsetzung mit der anderen Reaktionskomponente, z.B. Polyolen, Polyaminen oder Dämpfen von Diaminen aussetzt, beispielsweise nach Verfahren der DE—OS 3 039 146, JA 50—103 571, FR—PS 1 587 855, FR—PS 1 574 789, DE—OS 2 131 206 oder US—PS 2 955 056.

Man kann auch die Schaumstoffe einer quellend wirkenden Flüssigkeit aussetzen und bringt danach die Polyurethan-Reaktionskomponenten zur Einwirkung, wodurch Verhärtungen und Versteifungen des Schaumstoffes möglich sind und gegebenenfalls Einlagerungen in der gequollenen Schaumstoff-Matrix, z.B. nach Verfahren der FR—PS 1 341 717, 1 587 855, 1 574 789 oder der DE—AS 1 911 645, erfolgen. Solche Matrix-Schaumstoffe weisen weiterhin typische Schaumstoffeigenschaften, wenn auch andere Härte, Elastizitat oder chemische bzw. mechanische Eigenschaften aus.

Eine Vielzahl weiterer Patentschriften beschreibt die Verklebung bzw. die Verpressung von Schaumstoffteilchen (vorzugsweise Polyurethanweichschaumstoffabfallteilchen) mittels Polyisocyanaten, NCO-Prepolymeren und Polyolen, Polyaminen, Wasser oder anderen Reaktionspartnern, gegebenenfalls unter Zusatz von Kork, Fasern, Zellulosepulver, Flammschutzmitteln, Pigmenten, Metallpulvern oder Ruß zu neuartigen Verbundmaterialien, welche gegebenenfalls mit Überzügen oder mit Folien oder Metallplatten versehen oder damit verschweißt werden können. Derartige Verbundmaterialien werden beispielsweise als Isolier- und Dämmplatten, Auskleidungen, Matratzen oder Formkörper verwendet. Entsprechende Verfahren werden beispielswiese in den DE—OS 2 940 260, GB—PS 1 337 413, DE—OS 3 213 610, GB—PS 1 540 076, US—PS 4 254 177, DE—OS 2 908 161, DE—OS 3 120 121 und JA 57/028 180 beschrieben.

Technische Bedeutung hat bisher jedoch praktisch nur die Herstellung von Verbundblockschaum aus zerkleinertem Polyurethanschaumstoff, 10—20 Gew-% Isocyanatverbindungen, bis etwa 10 Gew.-% Füllstoffen und geringen Mengen Wasser erlangt. Der Füllstoff beteht dabei hauptsaehlich aus Farbpigmenten, um den Verbundschaum, der aus verschiedenfarbenen Schaumstoffchargen bestehen kann, eine einheitliche Färbung zu verleihen. Das bei der Herstellung des Verbundschaumstoffes verwendete Wasser bewirkt als Reaktionskomponente die Überführung der Polyisocyanatgruppen in Polyharnstoffgruppen unter Kohlendioxidentwicklung. Die Menge Wasser wird dabei so gewählt, daß sie etwa dem stöchiometrischen Bedarf der Isocyanate entspricht, höchstens aber nur in einem relativ kleinen Üaberschuß vorhanden ist, da sonst die Entfernung der Feuchtigkeit aus den 40—60 cm dicken Verbundblöcken Schwierigkeiten bereitet.

Als Rohstoffquelle für die als Matratzen oder elastische Teppichunterlagen verwendeten Verbundschaumstoffe relativ hoher Raumgewichte dienen vor allem die in großtechnischen Mengen beim Formschneiden von Blockweichschaum zwangsläufig anfallenden Verschnitte, die als Abfallpolyurethanschaumstoffe in so großen Mengen wohlfeil zur Verfügung stehen, daß nur ein Teil davon hierfür verwertet werden kann. So ist seit langem des Problem zu lösen, für die großen technischen Überschußmengen an Polyurethanabfallschaumstoffen eine ökologisch sinnvolle und wirtschaftlich vorteilhafte Verwendung zu finden, da beispielsweise eine Beseitigung durch Verbrennung oder Deponierung wegen der extrem großen Volumina technisch außerordentlich erschwert oder gar unmöglich ist.

Auch in der DE—OS 23 47 299 und DE—OS 25 21 265 werden homogene oder durch $CO_2$ schaumartig aufgetriebene, wassergequollene Polyurethan(harnstoff)-Gele beschrieben, allerdings ohne Verendung von vorgefertigten PU-Schaumstoffen, welche bis zu 50 Volumen-% Füllstoffe wie Silikate, Kieselsäuretypen, Aluminiumoxide, Zinnoxid, Antimontrioxid, Titandioxid, Graphit und graphierte Kohle, Retortenkohle, Ruß, pulverige Zementarten, Farbpigmente oder Fasern, sowie Zellulosepulver neben Tensiden oder Nährstoffen enthalten können. Auch hier beruht die Wasseraufnahme in den Gelen im wesentlichen in der Verwendung von hydrophilen Polyurethanen mit ≥40 Gew.-% Oxyethylensequenzen.

Erfindungsgemäß sollen jedoch stark hydrophile (wasseraufnahmefähige), vorzugsweise zusätzlich hohe Füllstoffgehalte aufweisende, mit Polyurethan(harnstoff)en modifizierte Schaumstoffpartikel hergestellt werden, welche in Wasser relativ stark gequollen sind und/oder in bzw. zwischen den Schaumstoff- und in den Füllstoff-Teilchen hohe Mengen an Wasser binden, so daß die Wasseraufnahmefähigkeitswerte (WAF) mindestens 30 Gew.-%, vorzugsweise ≥50 Gew.-% und bis 97 Gew.-%, bezogen auf wassergequollene Partikel (s. Meßmethode) betragen.

Es sind zwar auch schon wasserquellende Polyurethangele, welche gegebenenfalls 20 bis 80 Gew.-% Schleifmittel, z.B. Aluminiumoxid, Ceroxid, Wolframdioxid, Borcarbid, Siliciumcarbid, Carborundum, Asbestpulver oder Diamantpulver, sowie gegebenenfalls zusätzlich Graphit, Mikroglaskugeln, oder Glimmer, sowie Kurzfasern auf anorganischer oder organischer Basis, sowie Fungizide, Farbstoffe oder Farbpigmente enthalten, in der DE—OS 3 151 925 beschrieben worden. Derartige harte Füllstoffe sind jedoch nicht hydrofil und können auch nicht die anwendungstechnisch erwünschten Effekte der hohen Wasserbindungswerte zeigen, wie sie die erfindungsgemäßen, schaumstoffhaltigen und zusätzlich gegebenenfalls Füllstoffe, vorzugsweise überwiegend hydrophile, wasseraufnehmende Füllstoffe auf Basis von Braunkohlenstaub und/oder (Schwarz)Torf, bewirken.

In der DE—OS 3 103 499 werden im wesentlichen wasserfreie Polyurethangelmassen mit Polyolen als Dispersionsmittel beschrieben, welche Wirkstoffe und gegebenenfalls zusätzlich Farbstoffe, Pigmente, Fasern, anorganische Füllstoffe, Metallpulver, Aktivkohle, Zellulosepulver und Kieselsäurepräparate enthalten können. Solche polyolhaltigen Gele sind für den vorgesehenen Verwendungszweck ungeeignet, da sie in wäßriger Suspension zumindest einen großen Teil der dispergierten Polyole abgeben.

Es ist auch grundsätzlich die Einbettung von wachstumsfähigen Zellen in Polyurethan-Hydrogelen bekannt: siehe z.B. Tanaka et al., European Journal of Applied Microbiology and Biotechnology, 7, (1979) ab Seite 371. Auch in der DE—OS 29 29 872 wurde ein Verfahren zur Herstellung von hydrophilen, gelförmigen oder geschäumten Biokatalysatoren mit hoher Beladung an enzymatisch aktiver Substanz durch Polymereinschluß ganzer Zellen, von Zellfragmenten oder Enzymen, durch Mischen einer wäßrigen Suspension der enzymatisch aktiven Substanz mit hydrophilen Polyisocyanaten unter Bildung eines enzymatisch hochaktiven, hydrophilen Polyurethan-Netzwerks in Blockform oder perlförmiger Form beschrieben. Auf Seite 7 der genannten Offenlegungsschrift werden zum Stand der Technik auch weitere Veröffentlichungen genannt.

In den Polyurethangelen nach dem Stand der Technik müssen zwangsläufig hydrophile Polyurethane auf der Basis von Polyethern mit hohen Ethylenoxidanteilen aufgebaut werden, um eine ausreichende Waseraufnahmefähigkeit zu erreichen. Hierbei ergeben sich oft Reaktivitätsprobleme der (zumeist hohe Aktivitäten aufweisenden) hydrophilen Polyetherpolyole, Probleme bie der mechanischen Gelfestigkeit bei Verwendung hochhydrophiler Polyetherpolyole, abgesehen von dem Preis derartig aufgebauter hydrophiler Polyurethangelmassen, die zudem nur eine begrenzte Wasserlagerungsfähigkeit besitzen.

Überraschenderweise wurde nunmehr gefunden, daß man vorgefertigte Schaumstoffteilchen, vorzugsweise Polyurethanschaumstoffteilchen, insbesondere Polyurethanweichschaumstoffabfallteilchen, zur Herstellung hochgefüllter, stückiger, abriebfester, schaumstoffhaltiger Polyurethanharnstoffpartikel mit einem außerordentlich hohen Wasseraufnahmevermögen verwenden kann, wenn man Isocyanatverbindungen, vorzugsweise NCO-Prepolymere, welche hydrophilen oder hydrophoben Charakter zeigen, in Gegenwart von mindestens der Doppelten, beispielsweise 2- bis 60-fachen Gewichtsmenge, bevorzugt der 2- bis 30-fachen, insbesondere 5 bis 15-fachen Menge Wasser, bezogen auf Polyurethanschaumstoffgewicht, mit in Flocken- oder Folienform zerkleinerten Polyurethanschaumstoffteilchen und weiteren Füllstoffen, insbesondere Braunkohlenpulver und/oder Torf, vollständig reagieren läßt. Das Wasser hat bei der Herstellung der daraus resultierenden, hochgefüllten, abriebfesten Polyurethan(harnstoff)e nicht nur die Funktion als Kettenverlängerungsmittel unter Kohlendioxid- und Polyharnstoffbildung, sondern was ebenso wichtig ist, fungiert es in Riesenüberschußmengen angewandt gleichzeitig als Dipergiermittel, das bewirkt, daß die einzelnen vorgefertigten Schaumstoffteilchen die Isocyanatverbindungen und gegebenenfalls zugesetzte Füllstoffe über die gesamte zellulare Oberfläche gleichmäßig sowohl an der Oberfläche als auch an den inneren Zelloberflächen aufnehmen, ohne daß bei den Polyadditionsreaktionen die Schaumstoffteilchen selbst zu einem Verbundschaum verkleben, d.h. die Isocyanatverbindungen sind in Anwesenheit von sehr viel Wasser selektives Bindemittel nur für den Schaumstoff (und Füllstoff) in auf auf den Schaumstoffen. Durch die Mitverwendung von kationischen Gruppen bzw. kationenbilden Gruppen (z.B. tertiäre Aminogruppen-haltige) enthaltende Polyurethan-Reaktionskomponenten bzw. kationische Polymere wird sowohl eine hervorragende Benetzung der Schaumstoff- und gegebenenfalls Braunkohle- oder Torf-bzw. anorganischen Füllstoffteilchen bei der Reaktion erzielt, als auch in den schaumstoff- und füllstoffhaltigen Polyurethan(harnstoff)-Trägern die löslichen Anteile aus Braunkohle oder Torf (das sind vorzugsweise Huminsäuren) hervorragend zurückgehalten, so daß die erfindungsgemäßen Trägermassen in wäßriger Suspension kein Ausbluten von z.b. Huminsäuren zeigen, wie dies üblicherweise bei braunkohle- oder torfhaltigen Substanzen der Fall ist.

Erfindungsgemäß ist es möglich, aber nicht bevorzugt, bei der Polyurethan(harnstoff)-Bildung noch Biomassen zuzusetzen, und so evtl. lebende Bakterien, funktionsfähige Zellen, Zellteile oder auch Enzyme einzubinden.

Es war vom Fachmann nicht vorauszusehen, daß auf diese Weise mit Hilfe der Isocyanatverbindungen (NCO-Prepolymeren) überraschend große Mengen, bevorzugt etwa das den NCO-Prepolymeren entsprechende Gewicht oder gar bedeutend mehr, an Schaumstoffteilchen und weitere Füllstoffe abriebfest gebunden werden, dagegen weder der Füllstoff noch die Schaumstoffteilchen jeweils separat mit sich selbst zu Füllstoffagglomeraten oder zu einem Schaumstoffverbund verkleben. In vielen Fällen wird sogar die äußere Form des vorgefertigten Schaumstoffes weitgehend erhalten, jedoch die Wasserbindungseigenschaften für die vorgesehenen Erfindungszwecke verbessert.

Die erfindungsgemäß hergestellten, hochgefüllten, schaumstoffmodifizierten Polyurethan(harnstoff)-partikel weisen Eigenschaften auf, die in einem einzigen Reaktionsschritt (wie bei der Herstellung von Polyurethanschaumstoffen üblich) mit allen darin enthaltenden Komponenten nicht erreichbar wären. Besonders bemerkenswert ist, daß trotz beträchtlich hoher Füllstoffanteile in vielen Kompositionen die Elastizität der vorzugsweise eingesetzten, vorgefertigten Polyurethanweichschaumstoffe erhalten werden kann, gleichzeitig jedoch die dynamische Beanspruchbarkeit beträchtlich erhöht wird. Je nach Art des zusätzlich verwendeten Füllstoffes ist es auch möglich, das Raumgewicht drastisch zu erhöhen und weiteren Einfluß auf die Hydrophilie und Oberflächenaktivität der erfindungsgemäß hergestellten Produkte zu nehmen. Einer der wichtigsten, erfindungsgemäß erzielten Eigenschaften der neuen Polyurethanharnstoffpartikel ist ihre außerordentlich hohe Wasseraufnahmefähigkeit und ihre Abriebbeständigkeit, wenn diese füllstoffhaltige Polyurethanharnstoffpartikel in der besonders bevorzugten Verwendung als Träger für biologisch fermentative Synthesen, z.B. der Synthese von Penillin, eingesetzt werden sollen. Es war dabei besonders überraschend, daß auch hydrophobe NCO-Prepolymere bzw. hydrophobe Polyurethane zusammen mit den Schaumstoffen und zusammen mit weiteren Füllstoffen, insbesondere auf Braunkohle- und Torf-Basis, hervorragende Wasseraufnahmefähigkeitswerte (WAF) zeigen und eine geringe Tendenz zur Eigenverklebung der Partikel aufweisen. Sie sind daher gegenüber den hydrophilen PU-Gelen in vielen Fällen bevorzugt.

Gegenstand der Erfindung ist somit ein Verfahren zur Herstellung von schaumstoffhaltigen, Polyurethan(harnstoffen) aus NCO-Prepolymeren und Wasser, das dadurch gekennzeichnet ist, daß man

A) di- und/oder mehrfunktionelle NCO-Prepolymere, vorzugsweise mit einer Funktionalität > 2,1, besonders bevorzugt > 2,5, und einem NCO-Gehalt von 2 bis 12 Gew.-% NCO (vorzugsweise 2,5 bis 8 Gew.-%) auf der Basis von

a) zwei- und/oder mehrfunktionellen, höhermolekularen Verbindungen mit gegenüber Isocyanaten reaktiven H-Atomen mit Molekulargewichten von 400 bis 12 000 und mit einer Gesamtfunktionalität von ≥ 2,1, insbesondere ≥ 2,5, vorzugsweise Polyhydroxylverbindungen, insbesondere mehrfunktionellen Polyetherpolyolen,

b) 0 bis 5, vorzugsweise 0 bis 2 Mol pro Mol a) niedermolekularen, zwei- und/oder mehrwertigen Verbindungen des Molekulargewichts 32 bis 399, vorzugsweise 62 bis 254, und gegenüber Isocyanaten reaktiven H-Atomen, vorzugsweise Di- und/oder Polyolen des Molekulargewichts 62 bis 399,

c) gegebenenfalls kationische Gruppen aufweisenden oder kationische Gruppen bildenden Polyurethan-Ausgangskomponenten auf basis der höhermolekularen Komponente a) und/oder der niedermolekularen Komponente b) und/oder Zusätzen von kationische Gruppen tragenden oder kationische Gruppen bildenden Polymeren, vorzugsweise mit quarternären Ammoniumgruppen oder salzbildenden tertiären Aminogruppen, und gegebenenfalls zusätzlich Anionengruppen in höchstens dem Kationenäquivalent entsprechender Menge, bevorzugt aber nur kationische oder kationenbildende Gruppen tragenden Verbindungen,

d) überschüssigen Mengen bezogen auf a) bis c) an Di- und/oder Polyisocyanaten, vorzugsweise aromatischen Di- und/oder Polyisocyanaten, zur Ausbildung von NCO-Prepolymeren mit NCO-Gehalten von 2 bis 12 Gew.-% NCO (vorzugsweise 2,5 bis 8 Gew.-%),

B) gegebenenfalls mit Zusätzen von weiteren, niedermolekularen Polyisocyanaten zum NCO-Prepolymer A) in Mengen bis zum halben Gewichtsanteil von A) und bis zur Ausbildung von (A+B)-Mischungen mit NCO-Gehalten ≤ 30 Gew.-% NCO,

C) in überstöchiometrischen Mengen an Wasser, vorzugsweise einer mindestens 2-fachen bis etwa 60-fachen Gewichtsmenge, bezogen auf Feststoffgehalt von A + B, gegebenenfalls in Mischung mit Polyaminen, wobei diese vorzugsweise bis maximal 50 Äquivalent-% der NCO-Gruppen in A/B verwendet werden,

D) unter Zusatz von vorgefertigten, stückigen, zerkleinerten Schaumstoffen, insbesondere PU-Schaumstoffen und besonders bevorzugt PU-Weichschaumstoff(abfäll)en, vorzugsweise mit einem Raumgewicht von 10 bis 110 kg/m³ und Teilchengrößen von etwa 1 mm bis etwa 30 mm,

E) gegebenenfalls unter weiterem Zusatz von Braunkohle und/oder Torf, vorzugsweise Braunkohlenpulver und/oder feinteiligem Schwarztorf in Mengen von 1 bis 90 Gew.-%, vorzugsweise ≥ 20 Gew.-%, besonders bevorzugt ≥ 20 bis 70 Gew.-%, bezogen auf die füllstoffhaltigen Polyurethan-(harnstoff)-Massen,

F) gegebenenfalls in Gegenwart von weiteren anorganischen und/oder organischen Füllstoffen und üblichen Zusatzstoffen, Katalysatoren und Hilfsmitteln der Polyurethanchemie, wobei E) und/oder F) in einer Menge von mindestens 2 Gew.-% vorliegen,

4

G) und gegebenenfalls in Gegenwart von Biomassen unter Einbau von lebenden Zellen oder intakten Enzymen, zu füllstoffhaltigen, in wäßrigem Milieu abriebfesten, stark quellfähigen, nicht ausblutenden, schaumstoffhaltigen, vorzugsweise stückigen Polyurethan(harnstoff)-Massen mit einem Wert der Wasseraufnahmefähigkeit (WAF-Wert) von 30 bis 97 Gew.-%, vorzugsweise 80 bis 97 Gew.-%, bezogen auf wassergequollene, füllstoffhaltige Polyurethan(harnstoff)-Suspension ohne überstehendes Wasser, und einem Gehalt an kationischen Gruppen von 10 bis 3000, vorzugsweise 30 bis 1500 Milliäquivalenten kationischer oder kationbildender Gruppen pro 1000 g wasser- und füllstofffreier Polyurethan(harnstoff)-Masse, umsetzt.

Die bindende PU-Matrix, d.h. praktisch das Gewicht des eingesetzten NCO-Prepolymers beträgt dabei ≥ 5 Gew.-%, vorzugsweise ≥ 10 Gew.-% bei einer Obergrenze von ≤ 95 Gew.-%, vorzugsweise ≤ 75 Gew.-%, besonders beverziugt ≤ 65 Gew.-% des Gesamtfeststoffgehaltes an schaumstoff- und zusätzlich füllstoffhaltigen Polyurethan(harnstoff)-Massen.

Werden Füllstoffe neben den vorgefertigten Schaumstoffteilchen mitverwendet, so reduziert sich entsprechend diesem zusätzlichen Füllstoffgehalt der maximale Gehalt an Schaumstoffteilchen.

Mit zusätzlichen Füllstoffen F und/oer E neben den vorgefertigten Schaumstoffteilchen D) hält man im allgemeinen folgende Zusatzgrenzen ein:

| Zusatzstoffe | Mengengrenzen (Gew.-%) | vorzugsweise Mengengrenzen | besonders bevorzugte Mengengrenzen |
|---|---|---|---|
| D | 3—85 | 5—80 | 7—70 |
| E und/oder F | 2—90 | 20—70 | 28—60, |

wobei die Anteile an D bzw. E/F so ausgewählt werden, daß die Summe D, E, F

| ε D, E, F | 5—95 | 25—90 | 35—86 |

beträgt und somit die NCO-Prepolymeren A in folgenden Mengen eingesetzt werden:

| NCO-Prepolymere A (entspricht praktisch der bindenden PU-Matrix) | 5—95 | 10—75 | 14—65 |

Neben der vorgefertigten Schaumstoffteilchen D) werden in den angegebenen Mengen die Füllstoffe vom Typ E) und/oder Typ F) mitverwendet. Neben den Schaumstoffteilchen werden bevorzugt Füllstoffe E), d.h. insbesondere Braunkohlenstaub und/oder feinteiliger (Schwarz)Torf mitverwendet, da sie über ihr eigenes Wasserbindevermögen und die von ihnen verursachten Struktureigenschaften den Wasseraufnahmefähigkeitswert (WAF) besonders günstig zu erhöhen vermögen. Verwendet man dabei relativ hohe Mengen (≥ 20 Gew.-%) an Braunkohle und/oder Torf, so kann der Gehalt an vorgefertigten Schaumstoffteilchen beim Verfahren zur Herstellung entsprechender Polyurethanharnstoffpartikel sehr gering werden, z.B. ≥ 0,1 Gew.-% betragen. Wegen einer deutlichen Eigenschaftsmodifizierung bei der Verwendung der erfindungsgemäßen Massen, setzt man in der Praxis jedoch mindestens Gew.-% an vorgefertigten Schaumstoffteilchen ein und kombiniert diese mit einem Mindestgehalt an Braunkohle und/oder Torf von gleichfalls 5 Gew.-%.

Ein Teilmenge der Füllstoffe E) kann auch durch andere Füllstoffe F), z.B. anorganische oder organische Füllstoffe wie Steinkohlenstaub, Ruß, Aktivkohle, Fasern, Zellulosepulver, Korkpulver, Pigmente, Eisenoxide oder Metallpulver u.a. ersetzt werden, wobei zumeist untergeordnete Mengen (< 50 Gew.-%) an F) (gegenüber E)) eingesetzt werden. Diese Füllstoffe dienen, wie z.B. Seesand, einer Einstellung einer bestimmten Dichte, damit die Polyurethanharnstoffmassen nicht in Wasser aufschwimmen, oder sie dienen — wie bei Aktivkohle oder Aerosil — der Vergrößerung von inneren Oberflächen. Der Zusatz von Metallen oder Metallpigmenten, insbesondere Eisenoxiden wie Magnetit, fördert gegebenenfalls die Sauerstoffübertragung in biologischen Konversionsprozessen.

Erfindungsgegenstand sind auch die nach dem beanspruchten Verfahren zugänglichen, vorgefertigte Schaumstoffteilchen, zusätzlich Braunkohle und/oder Torf und/oder gegebenenfalls weitere Füllstoffe enthaltenden Polyurethan(harnstoff)-Massen mit hohen Wasseraufnahmefähigkeitswerten, gekennzeichnet insbesondere durch einen Schaumstoff- und Braunkohle- und/oder Torf-Gehalt bzw. weiteren Füllstoffgehalt von insgesamt ≥ 5 bis 95 bis Gew.-%, vorzugsweise 25 bis 90 Gew.-%, einen kationische Gruppen oder kationische Gruppen bildenden Gehalt von 30 bis 1000 Milliäquivalenten, vorzugsweise 50 bis 750 Milliäquivalenten pro 1000 g wasser- und füllstofffreier Polyurethanharnstoffmassen und einen Wasseraufnahmefähigkeitswert (WAF) von 30 bis 97 Gew.-% Wasser, vorzugsweise 80 bis 97 Gew.-%, bezogen auf wassergequollene, füllstoffhaltige Polyurethan(harnstoff)-Suspension.

Die erfindungsgemäßen, vorgefertigte Schaumstoffteilchen und zusätzlich Braunkohle- und/oder Torf-Füllstoffe und/oder weiteren Füllstoffe enthaltenden PU-Massen sind somit in einer (kationmodifizierten) Polyurethan-(harnstoff)-Matrix gebunden, wobei die füllstoffhaltigen Polyurethan(harnstoffe) zumeist in

5

feinteiliger oder stückiger Form, beeinflußt von der Form der vorgefertigten Schaumstoffteilchen, vorliegen.

Erfindungsgegenstand ist ferner die Verwendung der erfindungsgemäßen Massen als in Wasser gut suspendierbare, gegebenenfalls Biomassen eingebaut enthaltende Träger in Biokonversionsprozessen zur Herstellung organischer Verbindungen, als gegebenenfalls Pflanzenwuchsstoffe, Dünger oder Samen enthaltende Träger für Pflanzenwachstum mit hohem Wasserbindevermögen, als Filtriermittel für wäßrige Suspensionen oder als Adsorbentien für nicht wasserlösliche Flüssigkeiten wie z.B. (Roh)Oel oder Fette in Wasser.

Als vorgefertigte Schaumstoffteilchen können beispielsweise dienen: Polymerisate oder Copolymerisate von Ethylen, Styrol, Acrylnitril, (Methyl)-Butadien und Vinylverbindungen. Dies sind z.B. geblähte Polystyrolgranulate, expandiertes Polyethylen oder Schaumstoffabfälle auf Polymerisatbasis. Diese Schaumstoffe sind weniger bevorzugt und werden gegebenenfalls als Mischkomponente neben den bevorzugten Polyurethanschaumstoffen verwendet.

Als vorgefertigte (Polyurethan)Schaumstoffteilchen werden besonders bevorzugt die in größter Menge zwangsweise anfallenden Weichschaumstoffabfälle in zerkleinerter Form, z.B. als Mischung verschiedener Raumgewichte, die zwischen etwa 10—110 kg/m$^3$, vorzugsweise 12 bis 79 kg/m$^3$ schwanken und zumeist im Mittel zwischen 20—25 kg/m$^3$ betragen, eingesetzt. (Halb)Hartschaumabfälle sind für besondere Anwendungsfälle einsetzbar, im allgemeinen werden sie jedoch nur als Beimischung in feinteiliger, stückiger Form oder vorzugsweise in Pulverform als Füllstoffe eingesetzt. Gröbere (Halb)Hartschaumteile werden bei vielen Anwendungszwecken trotz Bindung durch Polyurethane zerrieben und zeigen dann mangelnde Beständigkeit.

Vorteilhaft verwendet man den beim Schneiden von Polyurethanhartschaumstoff-Blöcken zwangsweise in beträchtlichsten Mengen anfallenden staubfeinen Abfall. Hiermit bewirken schon sehr kleine Mengen eine in manchen Fällen gewüschte Veränderung der Eigenschaften der erfindungsgemäß hergestellten Produkte.

Als Schaumstoffabfall ist nicht nur Blockware verwendbar, sondern auch Formschaumaustrieb, dessen Verwertung wegen des hohen Anteils an glatter Außenhaut (Schwarte) bisher besonders problematisch war.

Das erfindungsgemäße Verfahren ist jedoch nicht alleine auf die Verwendung von Schaumstoffabfällen beschränkt. Es ist auch möglich, — wenn auch weniger bevorzugt — (Polyurethan)Weichschaumstoffe in Form endloser oder geschnittener Folie nachträglich mit einem sehr hohen Füllstoffanteil mit Hilfe zu Isocyanatverbindungen und großen Mengen Wasser zu Polyurethanharnstoffolien mit stark veränderten Eigenschaften im Vergleich zu den eingesetzten Folien umzusetzen. Die Dicke der Folien beträgt etwa 0,2—4 cm, bevorzugt 0,3—2 cm.

Die Hohlräume der Schaumstoffe werden bei der Bindung mit der Polyurethan-Matrix praktisch ganz oder zumindest teilweise gefüllt und somit steigt das Raumgewicht und die mechanische Festigkeit hinreichend stark an, damit die Schaumstoff-Flocken nicht mehr aufschwimmen und gegenüber mechanischen Einflüssen beständig sind. Es entspricht einer bevorzugten Form der Erfindung, daß man zusätzlich zu den (Leicht)-Schaumstoffen als Fuîllstoffpartikel feinteilige Torf- und besonders Braunkohlenstäube mitverwendet und dabei insbesondere solche Polyurethane als Matrix verwendet, welche kationisch (einschließlich kationbildend) modifiziert sind. Diese Träger können weiterhin als zusätzliche Füllstoffanteile in Mengen vorzugsweise unter 10 Gew.-% an anorganischen Füllstoffen, z.B. Aerosil, Seesand oder Eisenoxiden, enthalten.

Die erfindungsgemäß bevorzugt mitverwendeten Füllstoffe sind Torf, z.B. Weiß- oder Schwarztorf, und Braunkohle. Unter den Torfen ist der Schwarztorf bevorzugt. Der geeignetste Füllstoff für die Erfindung ist jedoch Braunkohle (in ihrer gemahlenen Form als sogenannter Braunkohlenstaub, der zumeist eine Restfeuchte von ≤ 60 Gew.-%, bevorzugt 3—20 Gew.-% Wasser enthält). Torf und Braunkohle sind wegen ihrer stark waserbindenden Eigenschaften außergewöhnlich wirksame Verbindungen, um die Hydrophilie von Polyurethan(harnstoff)-Trägern, überraschenderweise auch solchen auf Basis von hydrophoben Polyurethanausgangskomponenten, sehr wirksam zu erhöhen. Es hat sich gezeigt, daß sowohl durch die mechanische Struktur der Braunkohle oder des Torfes als auch durch ihre wasseraufsaugende Wirkung erfindungsgemäß ausgezeichnet zu verwendende Träger mit sehr hoher Wasseraufnahmefähigkeit entstehen. Am geeignetsten ist Braunkohlenstaub, der beispielsweise mit Gehalten an Kohlenstoff von etwa 68%, H von 5,3%, O von 25,7% und N von 1,0% (bezogen auf Trockensubstanz) ausgezeichnete Ergebnisse liefert. Dabei sind nur geringe Mengen an polyurethan-Matrix, z.B. 5 bis 20 Gew.-% notwendig, um abriebbeständige, füllstoffhaltige Polyurethan(harnstoff)-Trägermassen aufzubauen. Torf und Braunkohle vermögen hohe Mengen an Wasser zu binden, ohne das sich diese Materialien naß anfühlen — z.B. ≥ 150% Wasser, bezogen auf Braunkohle- oder Torf-Trockensubstanz. Selbst Braunkohlenstaub aus nativer Braunkohle enthält noch ca. 40 bis 60% Wasser. Mit Braunkohle oder Torf lassen sich in Gegenwart von PU-Schaumstoffen sehr hohe Füllstoffgehalte (d.h. Bindung mit einer nur sehr geringen Menge von Polyurethan-Matrix) erzielen. Getrocknete Braunkohle bzw. Torf mit Wassergehalten von etwa 3—20 Gew.-% zeigen vorteilhaft gute Bindung von Huminsäuren mittels der kationisch modifizierten Polyurethane. Die derart aus Kombinationen von Schaumstoffen und Braunkohle/Torf-Füllstoffen zugänglichen PU-Harnstoff-Träger zeigen besonders günstige wasserzurückhaltende Eigenschaften und ausgezeichnete Eignung als Träger in Biokonversionsprozessen.

6

In einer besonders bevorzugten Ausführungsform enthalten die schaumstoff- und füllstoffhaltigen Polyurethan(harnstoff)-Massen neben mindestens 5 Gew.-% Schaumstoffteilchen ⩾ 20 bis maximal 85 Gew.-% an Bruankohle und/oder Torf. Besonders bevorzugt sind Gehalte zwischen 20 und 70 Gew.-% an Braunkohle und/oder Torf. Besonders begünstigt sind solche erfindungsgemäßen Massen, welche an Schaumstoffen und Braunkohle oder Torf Mengen zwischen 35 und 80 Gew.-% enthalten. Ganz besonders günstig ist die Verwendung von Braunkohle als Zusatzstoff.

Neben den besonders bevorzugten Füllstoffen (Braunkohle und/oder Torf) können weitere, übliche Füllstoffe, bevorzugt in untergeordneten Mengen von weniger als der Hälfte der Braunkohle/Torf-Füllstoffmengen zugegeben werden.

Bei den Füllstoffen F) handelt es sich beispielsweise um organische Füllstoffe wie Aktivekohle, Holzkohlenpulver, Steinkohlenpulver, Kokspulver, Zellulosepulver und Korkmehl, ferner um feinteilige, über 100°C schmelzende organische Destillationsrückstände, insbesondere Destillationsrückstände der Toluylendiisocyanat-Destillation, welche beispielsweise durch Eintragen der Destillationsrückstände in Waser unter Denaturierung und anschließende Granulierung erhalten werden. Diese TDI-Rückstände können gegebenenfalls auch noch nachträglich durch Behandlung mit reaktive Wasserstoffe tragenden Verbindungen wie Ammoniak, Polyole oder Polyaminoverbindungen modifiziert sein. In vielen Fällen tragen sie noch geringe Mengen inkludierter NCO-Gruppen oder reaktive Abwandlungsprodukte von Isocyanaten. Derartige Destillationsrückstände werden beispielsweise in den DE—OS 28 64 814, 28 46 809, 28 46 815 beschrieben. Weiterhin geeignete Destillationsrückstände sind aber auch hochschmelzende Destillationsrückstände von Aminen, Phenolen, Caprolactam u.ä.

Die anorganischen Füllstoffe vom Typ F) wie Quarz, Seesand, pyrogene Kieselsäure (Aerosil), Silikate, Alumosilikate, Bentonite, Aluminiumoxid, Bimsstein, Kieselsole, Wasserglas, aber auch Calciumoxid, Calciumcarbonat, Diatomeenerde, Schwerspat, Gips, insbesondere aber auch feinteilige (Eisen-II- und/oder -III-)-Eisenoxide in Pigmentform oder als Magnetite werden bevorzugt — wie die organischen Füllstoffe F) — nur in Anteilen neben den Braunkohle/Torf-Füllstoffen vom Typ E) zugesetzt, um eine gewisse Regulierung der Oberflächenaktivität des spezifischen Gewichts der Träber zu gestatten, so daß diese in Wasser absinken oder schweben, jedoch keinesfalls aufschwimmen. Es ist jedoch auch möglich, die Füllstoffe vom Typ F) neben den Schaumstoffteilchen D) als alleinige Füllstoffe einzusetzen.

Als modifizierende Füllstoffzusätze können auch Fasern (z.B. anorganische Fasern) wie Glasfasern oder natürliche bzw. synthetische Fasern (z.B. Baumwollstaub) mitverwendet werden.

Die Korngröße der Füllstoffe liegt im allgemeinen zwischen 0,5 bis 1000 μm, bevorzugt unter 300 μm, besonders bevorzugt unter 100 μm, wobei insbesondere für Aktivkohle und anorganische Bestandteile sowie beim Kohlenpulver bzw. Holzkohlenpulver geringere Korngrößen bevorzugt werden als etwa bei dem erfindungsgemäß verwendeten Torf oder Braunkohlenstaub, da bei Torf und Braunkohle gegebenenfalls natürliche Faserige Anteile mit enthalten sein können.

Die Mengen an Zusätzen D, E und F wurden bereits angegeben. Die Anteile Füllstoffe werden jeweils in Gew.-%, bezogen auf die Trockensubstanz der gefüllten Polyurethan(harnstoff)massen berechnet. Die Obergrenze wird im allgemeinen von Zusammenhalt und der Abriebbeständigkeit der hochgefüllten Polyurethanmassen bestimmt.

Die Füllstoffe werden während der Bildung der Polyurethan(harnstoff)-Matrix, in verschiedenen, in der Beschriebung noch näher angegebenen Ausführungsformen eingebracht. So können sie einem der Ausgangsstoffe oder der vorpolymeren Produkte, beispielsweise den NCO-Prepolymeren oder den OHfunktionellen, höhermolekularen Polyolen, untergemischt werden und dann kann mit dieser Mischung die polyurethanbildende Reaktion durchgeführt werden. Bevorzugt werden jedoch die Schaumstoffe und/oder Füllstoffe zunächst mit Wasser benetzt oder angeteigt oder in Wasser dispergiert und mittels Zugabe der NCO-Prepolymeren von diesen umhüllt und gebunden, wobei jedoch gleichzeitig der Polyurethan(harnstoff)-Aufbau (insbesondere mittels Wasser als Kettenverlängerungsmittel) erfolgt.

A) NCO-Prepolymere

Als PU-bildende Ausgangskomponente für die Herstellung der NCO-Prepolymeren werden die an sich bekannten Ausgangskomponenten eingesetzt:

a) als höhermolekulare Polyhydroxylverbindungen mit Molekulargewichten von 400 bis 10 000, vorzugsweise 800 bis 8000, werden zwei- und/oder mehrfunktionelle höhermolekulare Polyole eingesetzt, welche vorzugsweise Funktionalitäten ⩾2,1, besser ⩾2,5 (und bis ca. 5), vorzugsweise ⩽ 3,5 aufweisen.

Als Polyhydroxylverbindungen sind Polyether vor den prinzipiell gleichfalls verwertbaren Polyestern, Polycarbonaten oder Polylactonen sehr bevorzugt, da sie auch im Langzeitverhalten wesentlich hydrolysenstabiler als die Estergruppen aufweisenden Polyhydroxylverbindungen sind.

Für den Aufbau von hydrophilen Polyurethanen sind solche Polyoxyalkylenether geeignet, welche eine größere Zahl, z.B. mehr als 20 Gew.-% oder ⩾ 30 Gew.-%, insbesondere ⩾ 40 Gew.-%, jedoch weniger als 85 Gew.-%, an Oxyethylen-Sequenzen enthalten. Die Oxyethylengruppen können endständig, statistisch verteilt oder bevorzugt segmentartig in den Polyethern eingebaut sein. Die Polyoxyalylenether können jedoch auch geringe Mengen an z.B. cycloaliphatischen oder aromatischen Gruppierungen enthalten, beispielsweise wenn sie auf cycloaliphatischen Polyolen oder aromatischen Verbindungen z.B. Dihydroxycyclohexanen oder Hydrochinon-bishydroxyethylethern .oder 4,4'-Dihydroxy-diphenyl-dimethylmethan gestartet sind. Auch aus höherfunktionellen Zuckeralkoholen oder Zuckern können durch Alkoxylierung geeignete Polyole aufgebaut werden.

Es können besonders überraschend jedoch auch hydrophobe Polyether zum Aufbau der PU-Matrix verwendet werden, z.B. Polyoxypropylenpolyole ohne oder mit geringen (z.B. $\leqq$ 20 Gew.-%) an eingebauten Oxyethylensegmenten. Überraschenderweise lassen sich auf der Basis Schaumstoffteilchen, Braunkohle und/oder Torf auch hiermit hohe Wasseraufnahmefähigkeitswerte (WAF) zeigende Trägersysteme aufbauen, die zumeist sogar eine verbesserte Langzeitbeständigkeit und besseres Sedimentationsverhalten in wäßrigen Biokonversionsmedien aufweisen und daher bevorzugt sind. Mit Steinkohlenstaub, Ruß oder Aktivkohle werden dagegen gefüllt Polyurethane erhalten, die nur Wasseraufnahmefähigkeitswerte, wie sie dem Schaumstoffgehalt entsprechen, zeigen.

Die Polyether auf Basis von Propylenoxid-Addukten sind die technisch bevorzugten Polyether; die Polyether können jedoch auch auf Basis anderer Alkyloxirane, z.B. Epichlorhydrin, Epoxybutane oder ihrer Mischungen mit z.B. Propylenoxid, in üblicher Weise aufgebaut werden.

Weiterhin sind auch Polyetheramine mit endständigen Endgruppen, z.B. durch Druckaminierung der sekundären Hydroxylgruppen oder Cyanethylierung und anschließender Reduktion erhältliche Polyether mit endständigen aliphatischen Aminogruppen, oder aliphatische oder bevorzugt aromatische Polyetheramine, welche durch Alkalihydrolyse von NCO-Prepolymeren entstanden sind, geeignete Ausgangsmaterialien.

Die höhermolekularen Verbindungen a) können auch bis zu 40 Gew.-% höhermolekularer Polyadditionsprodukte, z.B. aus Hydrazinhydrat und Toluylendiisocyanat, enthalten oder sogenannte Polymerpolyole darstellen, d.h. bis zu 40 Gew.-% Copolymerisate, gegebenenfalls Pfropf(co)polymerisate auf Acrylnitril- und (Meth)acrylesterbasis, enthalten.

b) Niedermolekulare, zwei- und/oder mehrwertige Vebindungen mit Molekulargewichten von 32 bis 399, vorzugsweise 62 bis 254, können in modifizierenden Mengen mit verwendet werden, vorzugsweise Di- und/oder Polyole oder Aminoalkohole, z.B. Ethylenglykol, 1,2-Propylenglykol, 1,4-Butandiol, 2,3-Butandiol, Neopentylglykol, 2-Methyl-propandiol-1,3, Hexandiol-1,6, Dodecandiol-1,12, besonders die relativ hydrophilen di-, tri-, tetra- und höhermolekularen Polyethylenglykole mit Molekulargewichten bis 399, aber auch Di-, Tri- und Tetrapropylenglykoldiole oder Di-, Tri- und Tetraoxymethylendiole. Als Aminoalkohole sind beispielsweise einsetzbar Bis-hydroxyethyl-amin, Bis-2-hydroxypropylamin, Aminozucker oder 2-Amino-propandiol-1,3.

Die Menge an b) ist Null bis etwa 5 Mol b) pro Mol a), d.h. der Zusatz von b) zu a) kann während der NCO-Prepolymerbildung auch unterbleiben. Trifunktionelle Polyole b) können zur Steuerung der Gesamtfunktionalität der NCO-Prepolymere eingebaut werden.

c) Beim Aufbau der hydrophilen und/oder hydrophoben Polyurethane werden kationische Gruppen oder kationische Gruppen bildende Gruppen im Polyurethan mit verwendet. Als kationische Gruppen können im Prinzip quarternäre oder salzartige Ammoniumgruppen, aber auch Sulfonium- oder Phosphoniumgruppierungen eingesetzt werden. Bevorzugt ist die Verwendung von quartären Ammoniumgruppen oder tertiäre Aminogruppen aufweisenden Verbindungen, welche letztere nachträglich in die Ammonium(salz)-Form überführt werden. Die Menge der einzubauenden kationischen Gruppen bzw. Kationen bildenden Gruppen beträgt 10 bis 3000 Milliäquivalente an Kationen bzw. Kationen bildenden Gruppen auf 1000 Gramm Feststoff-Polyurethan-Matrix. In den Fällen, wo man bereits quarternierte oder in Salzform überführte Verbindungen einsetzt, beträgt die Obergrenze im allgemeinen 2000 Milliäquivalente pro 1000 g Feststoff PU, da andernfalls während der Reaktion eine zu hohe Viskosität sich störend bemerkbar macht. Bevorzugt werden 30 bis 1500 Milliäquivalente kationischer oder Kationen bildender Gruppierungen, besonders bevorzugt sind 50 bis 750 Milliäquivalente kationischer oder Kationen bildender Gruppen auf 1000 Gramm Feststoff-Polyurethan eingebaut.

Als kationenbildende Verbindungen werden bevorzugt tertiäre Aminogruppen-enthaltende Di- oder Polyole verwendet, z.B. N-Methyl-di-(ethanol)amin oder -(propanol)amin, N,N-Dimethylaminomethyl-propandiol-1,3, Bis-hydroxyethylpiperazin, aber auch höherfunktionelle Verbindungen, wie z.B. Triethanolamin oder höhermolekulare Verbindungen wie z.B. Polyoxyalkylenether, welche auf tert. Amin-Polyole, z.B. de genannten Art, gestartet sind. Es können jedoch auch funktionelle, quarternierte Verbindungen wie z.B. Tetrahydroxyalkyl-ammoniumchloride oder -ammoniummethylsulfonate als Einbaukomponenten verwendet werden. Bisweilen ist es auch ausreichend, Verbindungen, welche tertiäre Amin-Endgruppen ergeben, in die Vebindungen einzubauen, z.B. N,N-Dimethylaminoethanol. Bei Verwendung solcher kationisch modifizierter Polyurethane (oder — weniger bevorzugt — anderer kationisch modifizierter Hochpolymerer) werden überraschenderweise die sonst wasserlöslichen Komponenten von Braunkohle oder Torf, nämlich die Huminsäuren und ähnliche saure, lösliche Verbindungen, quantitaiv fixiert. Somit wird auch für die Verwendung großer Mengen an Torf und/oder Braunkohle als Füllmittel in den hydrophilen Trägern bei Einbringen in Wasser eine farblose, völlig klare wäßrige Phase erhalten. Bisher stand der Verwendung von Torf oder Braunkohle in wäßrigen Systemen der gravierende Nachteil dieser wohlfeilen Naturstoffe im Wege, daß eine Braunfärbung des Wassers durch Herauslösen beträchtlicher Anteile einer ganzen Reihe von in Wasser bei pH 5 bis 9 direkt löslicher oder kolloidal in Lösung gehender Bestandteile, beispielsweise Huminsäuren oder deren Vorstufen, eintritt. Die kationisch modifizierten Polyurethane halten jedoch auch anorganische Bestandteile, z.B. Seesand, als Füllstoff sehr viel fester und abriebbeständiger gebunden.

Zur Herstellung von kationischen oder Kationen bildenden Polyurethan(harnstoff)massen ist am besten ein Weg über Isocyanatprepolymere geeignet, welche kationische Gruppen eingebaut enthalten oder eine zur Kationenbildung befähigte Gruppierung wie tertiär gebundenen Stickstoff, besitzen. Zur Salzbildung können übliche Säuren zugesetzt werden, z.B. Salzsäure, Phosphorsäure oder Schwefelsäure. In manchen Fällen reicht jedoch auch bereits die Salzbildung mit den Huminsäuren aus.

Es können anstelle von eingebauten oder einbaubaren kationischen Komponenten c) dem Reaktionsgemisch, z.B. in wäßriger Aufschlämmung der Schaumstoffe plus gegebenenfalls Braunkohle oder Torf-Füllstoffe, aber auch ersatzweise oder zusätzlich, kationische Polymere anderer Art, z.B. bevorzugt wäßrige Polyurethan-, aber auch Polyacrylat- oder Polyamid-Dispersionen, zugesetzt oder mitverwendet werden, doch ist dies eine weniger günstige Ausführungsform. In diesen Fällen der zugesetzten kationischen Dispersionen ist die vollständige Aufnahme in hochgefüllten Polyurethan(harnstoff)massen zumeist in nur relativ kleinen Mengen möglich — bei zu hohen Additivmengen wird sonst ein Teil davon im Wasser wieder ausgeschwemmt. Möglich, jedoch wenig bevorzugt ist die nachträgliche Applikation von wäßrigen, kationischen Dispersionen auf das nichtionische, hochgefüllte Polyurethanharnstoff-Trägermaterial, weil es in dieser nicht so stark fixierten Form oft als Flockungsmittel auf Substanzen in den Biokonversionsprozessen wirkt. Vorteilhafter ist dagegen die Zumischung dieser kationenaktiven Polyurethandispersionen durch Zumischung vor den Isocyanatreaktionen in wäßriger Phase, z.B. vor der Umsetzung des NCO-Prepolymers mit Wasser zum Polyurethan(harnstoff).

Neben den Kationengruppen können jedoch bis zur, oder besser unterhalb der Ionenäquivalenz, auch Anionengruppen (z.B. Sulfonatgruppen) im Polymeren oder als (polymere) Zusätze unter Ausbildung von Ampholytsystemen anwesend sein. Ein Überschuß von Anionen über die Kationengruppen ist bei Verwendung von Braunkohle oder Torf auf alle Fälle zu vermeiden, wenn die Huminsäuren im Polyurethan(harnstoff)-Träger gebunden bleiben sollen.

Die Kationengruppen in den hochgefüllten Polyurethan(harnstoff)massen haben nicht nur auf die Bindung der genannten fossilen Naturstoffe einen entscheidenden, günstigen Einfluß, sondern auch auf die Abriebbeständigkeit von Füllstoffen. Außerdem sorgen die Ionenladungen für eine feindisperse Verteilung oder sogar Lösung der Isocyanatverbindungen in den in sehr hohen stöchiometrischen Überschüssen angewendeten Mengen an Wasser (eine Art Emulgatorwirkung), so daß eine unerwünschte Koagulation der Polyurethane für sich selbst nicht auftritt, sondern eine sehr gleichmäßige Umhüllung der Schaumstoff- und Füllstoffteilchen durch den sich aus den NCO-Prepolymeren bildenden Polyurethan(harnstoff) erfolgt.

Überraschenderweise hat sich auch, gezeigt, daß anorganische Füllstoffe, wie Quarz, Seesand oder Bimssteinpulver durch die kationischen Polyurethane erheblich stärker und abriebfest gebunden werden und daß sich während der Polyurethanbildung keine Sedimentationserscheinungen der anorganischen Füllstoffe ergeben. Diese Zugabe von anorganischen Füllstoffen wird zumeist zur Regulierung des spezifischen Gewichtes der PU-(H)-Träger durchgeführt, um das spezifische Gewicht so einzustellen, daß kein Aufschwimmen der Träger während der biologischen Konversionsvorgänge in wäßrigen Lösungen eintritt. Extrem feinteilige anorganische Füllstoffe (0,1 bis 10 μm) erhöhen zusätzlich die spezifische Oberfläche der Trägermassen. Eisenoxide vermögen die Sauerstoffübertragung günstig zu beeinflussen. Die anorganischen Füllstoffe werden im allgemeinen jedoch nur in modifizierenden Mengen neben den anderen Füllstoffen, vorzugsweise neben Braunkohle und/oder Torf mitverwendet.

d) Als Polyisocyanate werden bevorzugt die di- und polyfunktionellen Polyisocyanate eingesetzt, z.B. Hexandiisocyanat, Dicyclohexylmethandiisocyanat, Isophorondiisocyanat, bevorzugt jedoch aromatische Di- und Polyisocyanate, wie die Toluylendiisocyanat-Isomeren, Diphenylmethan-4,4'- und/oder 2,4'- und/ oder 2,2'-Isomeren, sowie gegebenenfalls die höhermolekularen Polyphenylpolymethylenpolyisocyanate, wie sie durch Phosgenierung von rohen Formaldehyd-/Anilin-Kondensationsprodukten (Polyamingemischen) enstehen und als gegebenenfalls undestillierte Sumpfprodukte verwendet werden. Sulfongruppenhaltige Polyisocyanate sind gleichfalls als hydrophile Polyisocyanate einsetzbar.

Als Polyisocyanate können jedoch weiterhin alle bekannten Polyisocyanate zur Umsetzung benutzt werden, wie sie beispielsweise in der DE—OS 28 32 253 ausführlich aufgeführt werden. In der letztgenannten DE—OS werden auch die Beispiele für a, b, und c) genannt.

Die reaktiven Komponenten a), b) und c) werden mit überschüssigen Mengen an Di- und/oder Polyisocyanaten zur Ausbildung von NCO-Prepolymeren A) mit NCo-Gehalten von 2 bis 12 Gew.-% NCO, vorzugsweise 2,5 bis 8 Gew.-%, insbesondere 2,5 bis 6 Gew.-% NCO, in üblicher Weise z.B. gemeinsamem Erwärmen der Komponenten bei 50 bis 100°C bis zur NCO-Prepolymerbildung, umgesetzt.

Die Gesamtfunktionalität der NCO-Prepolymeren A) soll mindestens 2,1, vorzugsweise ≥ 2,5 betragen, d.h. mindestens eine der Komponente a), b) und/oder c) muß mehr als bifunktionell sein.

Diese NCO-Prepolymeren A) können gegebenenfalls mit weiteren Mengen an niedermolekularen Polyisocyanaten B) in Mengen bis zum halben Gewichtsanteil von A) und bis zur Ausbildung von A) + B)-Mischungen mit NCO-Gehalten von ≤ 30 Gew.-% NCO, vorzugsweise ≤ 20 Gew.-% NCO, vermischt werden. Es können dabei auch unterschiedliche Polyisocyanate zur Prepolymerbildung d) und zuer weiteren Aufstockung B) des NCO-Gehaltes verwendet werden. Bei gleichen Polyisocyanaten kann auch bereits bei der Prepolymerbildung A) ein entsprechend hoher Anteil an Polyisocyanaten d) verwendet werden.

C) Die Umsetzung der NCO-Prepolymeren A) oder (A + B) erfolgt mit weit überstöchiometrischen Mengen an Wasser im heterodispersen System. Bevorzugt verwendet man die Wassermenge zum Anteigen oder Dispergieren der Schaumstoffe und gegebenenfalls Füllstoffe.

Die NCO-Prepolymeren werden dann — gegebenenfalls selbst bereits in anteiligen Wassermengen dispergiert —, zugemischt, wobei die NCO-Prepolymere im allgemeinen die Schaumstoffe und Füllstoffe gut benetzen, umhüllen und dann mit Wasser relativ langsam (bei zugesetzten Mengen an Di- oder Polyaminen schneller) zur Polyurethan(harnstoff)-Matrix aushärten. Durch Erhöhung der Reaktionstemperatur kann die Wasserreaktion auf wenige Minuten verkürzt werden.

F) Als weitere Zusatz- und/oder Hilfsstoffe können alle in der PU-Chemie üblichen Stoffklassen eingesetzt werden, z.B. Stabilisatoren, UV-Absorber, Dispergiermittel, Emulgatoren, Silikonderivate, Farbstoffe und Pigmente. Als Katalysatoren können die üblichen Polyurethankatalysatoren, wie tert. Amine, Metallkatalysatoren oder Zinnkatalysatoren eingesetzt werden, was in vielen Fällen jedoch nicht zwingend erforderlich ist.

Die hydrophilen, (PU)-Schaumstoff- und vorzugsweise zusätzlich Braunkohle- und/oder Torf-haltigen Polyurethan(harnstoff)-Träger liegen bei Verwendung stark hydrophiler NCO-Prepolymeren in Form einer mehr oder minder gelartigen, gequollenen, gegebenenfalls schaumgelartigen Form vor, die sich feucht anfühlen. Erfindungsgemäß hergestellte Trägermassen auf Basis hydrophober NCO-Prepolymerer weisen dagegen einen trockenen Griff auf und unterscheiden sich dadurch deutlich von den gelartigen Produkten. Sie zeigen eine gute Abriebfestigkeit. Trotzdem zeigen auch sie überraschend hohe Wasseraufnahmefähigkeits(WAF) -Werte. Sie fallen auch — im Gegensatz zu Gelen — direkt in kleinstückiger, direkt verwertbarer Form an. Sie sind daher erfindungsgemäß bevorzugt. In manchen Fällen ist auch der Einsatz einer Mischung von hydrophilen und hydrophoben NCO-Prepolymeren von großem Vorteil, vorallem im Hinblick auf das direkte Anfallen in stückiger Form. Sie können prinzipiell auch nach allen Verfahren und aus Ausgangskomponenten hergestellt werden, wie sie beispielsweise in den DE—OS 23 47 299, DE—PS 25 21 277, DE—PS 25 21 265, DE—OS 31 03 500, DE—OS 31 03 564 und DE—OS 31 51 925 beschrieben sind.

Ein bevorzugter Weg zur Herstellung der Polyurethan(harnstoff)-Träger liegt in der Herstellung über NCO-Prepolymere (A), welche aus hydrophoben oder hydrophilen, höhermolekularen Polyhydroxyl- oder Polyaminverbindungen a), vorzugsweise gegebenenfalls Oxyethylengruppen-haltigen Polyetherpolyolen, gegebenenfals Kettenverlängerungsmitteln b), kationische Gruppen oder kationische Gruppen bildende Gruppen enthaltenden Verbindungen c) und überschüssigen Mengen Polyisocyanat d) hergestellt werden.

Durch Reaktion mit (überschüssigen Mengen an) Wasser tritt Reaktion zum Polyurethan(harnstoff) ein. Das Wasser kann gegebenenfalls geringere Mengen von bis etwa 50 Äquivalent-% der NCO-Gruppen von A) an Di- und Polyaminen enthalten, wobei eine sehr schnelle Teilverfestigung zu einem Polyurethanharnstoff erfolgt.

Die Suspendierung oder Benetzung der Schaumstoffe und gegebenenfalls Füllstoffe erfolgt bei der diskontinuierlichen Herstellung vorzugsweise zuerst mit der überschüssigen Menge, danach wird das NCO-Prepolymere zudosiert.

In geeigneten, kontinuierlich arbeitenden Mischaggregaten, wie einem Doppelpaddel-Schneckentrog, können alle Komponenten praktisch gleichzeitig oder nur wenige Sekunden nacheinander zugegeben und intensive vermischt werden, wobei die Isocyanatreaktionen sofort beginnen. Auf die Rektionsgeschwindigkeit kann man in bekannter Weise mit Hilfe von Katalysatoren und/oder Anwendung erhöhter Temperaturen Einfluß nehmen. Zum Start der Isocyanatreaktion werden Temperaturen zwischen 10 bis 90°C, bevorzugt 20 bis 70°C gewählt. In vielen Fällen sind die üblichen Raumtemperaturen gut geeignet. Nach einer vollständigen Benetzung der Füllstoffe kann die Reaktionstemperatur bis auf etwa 90°C gesteigert werden.

Wasser ist nicht nur, wie bereits erwähnt, als Reaktionspartner für die Polyisocyanatverbindungen erforderlich, sondern es dient auch als Dispersionsmittel in größeren Überschußmengen, gleichgültig, ob hydrophile oder hydrophobe Isocyanatverbindungen eingesetzt werden.

Besonders wichtig ist die resultierende Hydrophilie, d.h. die Wasseraufnahmefähigkeit und das Wasserrückhaltevermögen des Endproduktes, d.h. des hochgefüllten, Schaumstoffteilchen und gegebenenfalls weitere Füllstoffe enthaltenden, hydrophilen Polyurethan(harnstoff)-Trägers, die Abriebfestigkeit dieses hydrophilen Trägers, eine möglichst jahrelange Beständigkeit und Unlöslichkeit im wäßrigen System und eine leichte Sinktendenz oder mindestens Schwebefähigkeit in der wäßrigen Phase, d.h. eine nicht aufschwimmende Charakteristik der Teilchen.

Eine maximale Hydrophilie ist entweder dadurch zu erreichen, daß alle in dominierender Menge verwendeten Komponenten stark hydrophil sind, z.B. Polyurethan(harnstoff)-Vorprodukte wie Polyole oder Kettenverlängerungsmittel oder auch der (in bevorzugt etwa gleicher oder besser größerer Menge als die Polyurethane verwendete) hydrophile Füllstoff. Eine zu stark hydrophile Polyurethanmatrix ist jedoch hinsichtlich der Langzeitlagerung im Wasser meistens nicht ausreichend beständig und im Wirbelbett nicht abriebfest. Bessere Beständigkeit erzielt man mittels Einbau der stark Wasser-bindenden (PU-Weich)-Schaumstoffe, bevorzugt unter zusätzlicher Mitverwendung von hydrophilen, feinteiligen Braunkohle/Torf-Teilchen in einer hydrophoben, kationengruppen-haltigen/-bildenden PU-Matrix.

Die Wasseraufnahmefähigkeit der erfindungsgemäß zu verwendenden Träger ist in unterschiedlichen Ausführungsformen der Herstellung bzw. Zusammensetzung zu erzielen, doch gilt als Regel zum Handeln, daß ein wesentlicher Anteil der Wasseraufnahmefähigkeit (WAF-Wert) durch die eingebrachten Schaumstoffteile und vorzugsweise zusätzlich durch die fossilen Zellulosen, d.h. insbesondere Schwarztorf und am günstigsten Braunkohle(nstaub), bewirkt wird und die Polyurethan(harnstoff)-Vorprodukte in möglichst geringer Menge und hoher Bindungskraft eingesetzt werden.

Auch die bei der Reaktion mit den NCO-Prepolymeren und Füllstoffen vorhandene Wassermenge ist von größer Bedeutung:

Bei Verwendung von relativ geringen Mengen Wasserüberschuß, beispielsweise 20 Teile Wasser auf 80 Teile NCO-Prepolymer plus Füllstoffe entsteht mit z.B. hydrophoben NCO-Prepolymeren ein feinpulvriges oder krümeliges, nicht ausreichend hydrophiles und somit ungeeignetes Produkt mit hoher Ausschwemmrate; erst mit einer deutlich höheren Wassermenge bindet das NCO-Prepolymer die Füllstoffe zu einem abriebfesten, wasseraufnahmefähigen Träger mit den erfindungsgemäß erforderlichen Eigenschaften, indem das NCO-Prepolymer (insbesondere bei entsprechend hoher kationischer Modifizierung) die Schaumstoff- und Füllstoffteilchen erst ausreichend benetzend umhüllt und dann zum Polyurethan(harnstoff) abreagiert und dabei die Schaumstoffteilchen und auch die bevorzugten Braunkohlen- oder Torf-Teilchen fest, aber wasserdurchlässig einschließt.

Zur Prüfung auf die gewünschten Eigenschaften der Träger unterzieht man etwa 30 bis 300 g große Vorproben der in Betracht gezogenen, hochgefüllten Polyurethan(harnstoff)-Träger, die man durch systematische Variation der Art und Menge der Isocyanatkomponenten und der Füllstoffe in Anwesenheit variabler, aber immer großer Überschußmengen Wasser in kleinen Vorversuchen hergestellt hat, einem Test in mit Wasser gefüllten Säulen, z.B. mit einem Durchmesser von 10 bis 20 cm, durch die man von unten durch eine Fritte oder eine feingelochte Siebplatte Luft strömen läßt. Innerhalb von 24 Stunden läßt sich so der Abrieb, die Sinktendenz, die Farbe und die Transparenz der wäßrigen Phase leicht ermitteln. Die Anwesenheit und richtige Dosierung von Überschußmengen Wasser ist, wie dargelegt, bei der Herstellung des Trägermaterials für die vorgesehene Verwendung der gefüllten Polyurethan(harnstoffe) als Bioaktivatoren in biologischen Konversionsprozessen von großer Bedeutung und kann nach den gegebenen Hinweisen durch einfache Vorversuche optimiert werden.

Die Formgebung bei der Herstellung der hochgefüllten Polyurethan(harnstoffe) wird in vielen möglichen Varianten auf die jeweilige Verfahrenstechnik der vorgeschlagenen Verwendung abgestimmt. Regelmäßiges oder umregelmäßiges stückiges "Granulat" erhält man entweder direkt oder nach den üblichen Schneide- oder Granuliertechniken als block-, strang- oder bandförmige Ware. In speziellen Fällen werden die erfindungsgemäß einzusetzenden, hochgefüllten Polyurethan(harnstoffe) in die Bioreaktoren in Folienform gehängt oder spiralartig gewunden eingesetzt. In diesen Fällen werden zur Stabilisierung besonders großer Flächen gegebenenfalls textile Unterlagen nach an sich bekannten Gieß- oder Rakelverfharen mitverwendet.

In der kostengünstigsten und einfachsten Ausführungsform werden die Träger in Form eines unregelmäßigen Granulats in einer Größe von 0,1 bis 10 cm, bevorzugt 1 bis 3 cm eingesetzt. Dazu werden die weitgehend oder vollständig ausreagierten, hochgefüllten Polyurethan(harnstoff)e gegebenenfalls in vorgefertigter Strang-, Block- oder Bandform mit üblichen Zerhackern oder Schneidgranulatoren auf die geeignete Stückgröße zerkleinert. Eventuell entstandenes Feinkorn kann beim Waschen gegebenenfalls isoliert und abgetrennt werden.

Werden die Isocyanat-Reaktionen diskontinuierlich in Knetern oder in mit Pflugschar- oder Paddeln ähnlichen Werkzeugen ausgerüsteten Mischapparaturen durchgeführt, ist eine nachträgliche Zerkleinerung meistens nicht mehr notwendig.

Die erfindungsgemäßen, stark wasserhaltigen und/oder wassergequollenen, hydrophilen Trägermassen stellen im allgemeinen weichelastische, sich mehr oder weniger feucht anfühlende, abriebfeste Teilchen dar, welche sich in Wasser suspendieren lassen und dort langsam absinken. Im Falle einer rein hydrophilen PU-Matrix sind die Trägermassen als solche weniger wassergequollen als wasserbindend (z.B. über die Schaumstoffstruktur und gegebenenfalls hydrophilen Füllstoffe).

Es war nicht vorauszusehen, daß die überwiegend mit leichten Schaumstoffen und vorzugsweise zusätzlich mit Füllstoffen wie Braunkohle oder Torf hochgefüllten, Polyurethan(harnstoff)massen als Träger mit stark hydrophilen (wasseraufnahmefähigen) Eigenschaften in homogener, zumeist stückiger Struktur ausreichend abriebfest hergestellt werden können, auf die Biokonversionen einen so günstigen Einfluß nehmen, obwohl die Füllstoffe aktiver Art, wie z.B. Schwarztorf und Braunkohle, innerhalb der Polyurethanmasse eingebettet liegen, die vorzugsweise sogar hydrophober natur sein kann.

Die erfindungsgemäß eingesetzten Träger eignen sich für viele der gebräuchlichen Biokonversions-verfahren, z.B. zur Herstellung von Zitronensäure aus Stärke, zur Hydrolyse von Penicillin G mittels Acylasen zu 6-Aminopenicillansäure, ferner zur Herstellung von stereospezifischen, biologisch aktiven Verbindungen, oder zur Vergärung von zuckerhaltigen Wässern in der Rübenzuckerindustrie.

Der im Polyurethan inkorpierte Schaumstoff und gegebenenfalls Füllstoff hat in mehrfacher Hinsicht einen vorteilhaften Einfluß auf das verbesserte Biokonversions-Verfahren. Je nach Art der Schaum- und Füllstoffe und Art der Polyurethan-Matrix werden die mechanische Festigkeit und die Hydrophilie des Polyurethans verbessert und insbesondere überraschend die bioaktive Assimilationsfähigkeit der umzuwandelnden organischen Stoffe wesentlich erhöht. Außerdem ist der im Polyurethan Träger eingebundene Füllstoff oder die Füllstoffmischung gleichzeigt ein Regulator für die Einhaltung optimaler, spezifischer Gewichte der für Wasser durchlässigen, erfindungsgemäß eingesetzten Träger, so daß in den üblichen Gefäßen eine gleichmäßige Verteilung der Träger mit schwacher Sinktendenz oder die Erhaltung eines schwebenden Zustandes möglich ist. Dies ist für die meisten Prozesse von besonderer Bedeutung oder sogar eine verfahrenstechnische Voraussetzung.

Auf jeden Fall ist in den so resultierenden, hochgefüllten Polyurethan(harnstoff)massen der beispielsweise als Füllstoff eingesetzte Polyurethan-Weichschaumstoff-Abfall mit einem mittleren Raumgewicht von nur 23 kg/m$^3$ in seiner ursprünglichen Struktur und physikalischen Eigenschaft völlig verändert und wird erst durch die grundlegende Modifizierung mit den Polyurethanen erfindungsgemäß verwendbar. In den zumeist teilgefüllten Hohlräumen des Schaumstoffes kann eine hinreichende Menge Wasser gebunden werden, so daß es möglich ist, Weichschaumstücke als Füllstoff in Kombination mit sowohl hydrophoben wie auch hydrophilen Polyurethan-Reaktionskomponenten, vorzugsweise NCO-Voraddukten und vorzugsweise mit zusätzlich hydrophilen Füllstoffen (z.B. Braunkohle) zu verwenden. Durch die Wechselwirkung von hydrophilen Füllstoffen (vorzugsweise Torf oder Braunkohle) und den physikalisch gebundenen Wassermengen bildet sich eine vorteilhafte Struktur aus, auf der auch ein Bakterienbewuchs in die restlichen Hohlräume der modifizierten Schaumstoffe auftreten kann. Zur Regulierung des spezifischen Gewichts können bei der Herstellung dieser hochgefüllten Polyurethan(harnstoff)massen auch anorganische Füllstoffe in feinstzerteilter Form verwendet werden, wodurch das notwendige spezifische Gewicht für das Nichtaufschwimmen in den wäßrigen Reaktionsflüssigkeiten, bei z.B. Biokonversionsprozessen, erreicht wird.

Der Grad der Hydrophilie wird bei den erfindungsgemäßen, hochgefüllten Polyurethan(harnstoff)massen bevorzugt so eingestellt, daß eine hohe Wasseraufnahme innerhalb von Stunden oder wenigen Tagen unter starker Quellung erfolgt oder eine größere Wassermenge bereits bei der Herstellung er Polyurethanmassen als disperse Phase vorliegt und somit die Träger bereits voll gequollen sind.

Der "in situ"-Einbau von Mikroorganismen in Polyurethane oder andere Kunststoffe ist, selbst unter sehr schonenden und technisch aufwendigen Bedingungen, nicht ohne wesentlichen Verlust an vermehrungsfähigen Bakterien und starker Minderung der Bioaktivität möglich. Die Herstellungsbedingungen sind dabei insbesondere hinsichtlich der Temperatur (etwa +10°C) anzupassen. Trotzdem ist dieses, an sich mögliche Verfahren nicht bevorzugt und zumeist auch nicht notwendig, da ein Aufwachsen von Biomassen in die Schaumstoff- und Braunkohle- oder Torf-haltigen Polyurethan(harnstoff)-Trägermassen in hervorragender Weise erfolgt.

Die Verwendung der erfindungsgemäßen, bevorzugt Schaumstoffe- und Braunkohle- oder Torf-haltigen sowie kationischen Polyurethan-(harnstoff)-Trägermassen liegt in ihrer hochhydrophilen Eigenschaft und ist gegebenenfalls in ihrer leicht porigen Struktur begründet. Man kann sie als Bodenverbesserer oder Spezialwachstumsträger hydrophiler, leicht bewurzelungsfähiger Art für Pflanzen benutzen, da sie beliebige Pflanzennährstoffe enthalten können, einen sehr lange verwertbaren Wassergehalt und gegebenenfalls Düngergehalt aufweisen und leicht wieder rückquellen.

Den Trägermassen können bei der Herstellung auch Samen zugemischt werden, die dann zum Keimen gebracht werden und in z.B. Plattenform, beispielsweise als Petersilienrasen, verwertbar sind oder in kleinstückiger Form als Setzlinge mit PU-Trägerstücken eingesetzt werden können.

Eine weitere, wichtige Verwertung liegt in ihrer Verwendung als Träger für Bakterien oder Enzyme in Biokonversionsprozessen zur Herstellung komplizierter organischer Verbindungen. Die stückigen Träger können von den Reaktionsbrüden oder Fermentationsbrüden durch Filtration leicht abgezogen werden. Die stückigen Träger können auch als Filtriermedium für feinteilige Verunreinigungen benutzt und, beispielsweise durch Rückspülen, regeneriert werden. Eine besonders wirkungsvolle Verwendung finden die erfindungsgemäßen Träger als Adsorbentien für (Roh)Oel oder andere, nicht wasserlösliche organische Flüssigkeiten.

Ausführungsbeispiele

A) Allgemeine Verfahrensweise zur Herstellung der Trägermassen

A1. Herstellung der NCO-Prepolymeren

Die Herstellung der NCO-Prepolymeren erfolgt in an sich bekannter Weise in einer Rührapparatur durch etwa 2- bis 3-stündiges Erhitzen der Ausgangskomponenten (höhermolekulare Polyhydroxylverbindung, gegebenenfalls niedermolekulare Polyole, vorzugsweise tertiären Stickstoff enthaltende Polyole und Polyisocyanate) bei Temperaturen von etwa 70 bis 90°C, bis annähernd der berechnete NCO-Gehalt erreicht ist. Zusammensetzung siehe Tabelle 1).

Im Falle der Herstellung basischer NCO-Prepolymere, die nicht kurzfristig weiter umgesetzt werden, kann deren Lagerstabilität mit sehr kleinen Mengen (z.B. 0.05—0.15 Gew.-%, berechnet auf Prepolymer) an Mineralsäuren (z.B. Schwefelsäure oder Phosphorsäure) wesentlich (z.B. um mehrere Monate) verlängert werden.

Tabelle 1  Zusammensetzung und Charakterisierung der NCO-Prepolymeren (PP)

| Typ | fortlau-fende Bezeich-nung | Viskosität mPas/25°C | % NCO | Isocyanat Menge/Art | Polyetherpolyol Menge / Art | NM | DMS | IQU |
|---|---|---|---|---|---|---|---|---|
| KIPP | A | 12.500 | 5,1 | 16,6 TDI | 80,4 PHILV | 1,5 | 1,5 | 120 |
| KIOPP | B | 64.000 | 5,3 | 19,1 TDI | 57,1 PHILV<br>19,0 PHOBL | 2,4 | 2,4 | 200 |
| BIPP | C | 11.200 | 5,6 | 18,5 TDI | 79,1 PHILV | 2,4 | 2,4 | 200 |
| IPP | D | 56.000 | 4,9 | 22,4 D44R | 77,6 PHILV | – | – | – |
| KOPP1 | E | 9.500 | 5,9 | 20,0 TDI | 41,9 PHOBV<br>35,0 PHOBL | 1,5 | 1,5 | 120 |
| KOPP2 | F | 12.200 | 3,2 | 14,1 TDI | 45,9 PHOBV<br>38,4 PHOBL | 0,8 | 0,8 | 60 |
| (K)OPP | G | 15.600 | 5,7 | 20,6 TDI | 76,3 PHOBV | 3,1 | | 260 |
| OPP | H | 7.800 | 3,3 | 11,9 TDI | 88,1 PHOBV | – | – | – |
| OPP | I | 18.300 | 3,6 | 7,7 TDI<br>12,2 D44R | 80,1 PHOBV | – | – | – |
| (K)OPP | K | 4.460 | 5,6 | 29,4 TDI | 42,5 PHOBV<br>35,5 PHOBL | 1,5 | – | 130 ST |

Typ = Charakterisierung der NCO-Prepolymeren (PP)
K   =       Kationisch
(K) =       Basisch mit tert. Stickstoff, zur Kationenbildung befähigt
I   =       Hydrophil
O   =   .   Hydrophob
(Mengenangaben in Gewichtsteilen)
ST  =       Langzeitstabilisierung durch Zugabe von 0,1 Gew.-% conc. $H_2SO_4$ (in Form einer
            10 %igen Lösung in Polypropylenetherdiol (Mol.-Gewicht 2000).

Die Mengenangaben in der Tabelle 1 erfolgen in Gewichtsteilen. Eingesetzte Isocyanate:

TDI = Toluylendiisocyanat-2,4-, -2,6-Isomerengemisch 80:20

D 44 R = Destillationsrückstand aus der Produktion von 4,4'-Diphenylmethandiisocyanat, Anteile an höhermolekularen Polyphenylpolymethylen-polyisocyanaten enthaltend, NCO-Gehalt 29,8 Gew.-%.

Polyetherpolyole:

PHILV = hydrophile, verzweigter Polyether, gestartet auf Trimethylolpropan, umgesetzt mit 40 Teilen Propylenoxid und 60 Teilen Ethylenoxid, OH-Zahl 26.

PHOBV = hydrophober, verzweigter Polyether, gestartet auf Trimethylolpropan mit 80 Teilen Propylenoxid und danach 20 Teilen Ethylenoxid umgesetzt, OH-Zahl 28.

PHOBL = hydrophober, linearer Polyether aus 1.4-Butandiol und Propylenoxid, OH-Zahl 56.

Verbindungen mit tertiarem Stickstoff:

NM = N-Methyl-diethanolamin

Quarternierungsmittel:

DMS = Dimethylsulfat

PPS = 85 %ig Polyphosphorsäure

Ionifizierungsangaben:

IQU = Kationenäquivalent oder tertiäres Stickstoffäquivalent (als Kationen bildende Gruppe) in Milliäquivalenten pro 1000 g NCO-Prepolymer.

A)2. Umsetzung der NCO-Prepolymeren zu den Trägermassen entsprechend Patentbeispielen (allgemeine Verfahrensvorschrift zur diskontinuierlichen Herstellung)

Die vorgefertigten Schaumstoffteile und gegebenenfalls Füllstoffe werden in der angegebenen Menge Wasser suspendiert oder das Wasser in die Schaumstoffe/Füllstoffe eingerührt und dann das NCO-Prepolymer schnell und intensive bei Raumtemperatur eingemischt. Im Falle der stark hydrophilen NCO-Prepolymeren erstarrt das Reaktionsgemisch bei Raumtemperatur schon nach wenigen Minuten, z.B. 1 bis 3 Minuten, bei den hydrophoben NCO-Prepolymeren jedoch erst nach 1 bis 2 Stunden. Die Reaktionszeit wird gegebenenfalls durch Zugabe von 0,1 bis 0,5 Gew.-% Katalysator, bezogen auf Prepolymermenge, und/oder durch Verwendung von heißem Waser (ca. 80°C) auf wenige Minuten verkürzt. Die Umsetzung erfolgt in üblichen Rührapparaturen (bei Laboransätzen); für technische Mengen werden vorzugsweise mit pflugscharähnlichen Mischwerkzeugen ausgerüstete, horizontal aufgestellte, konventionelle Mischgeräte eingesetzt.

Soweit die Kationenbildung im Prepolymer noch nicht erfolgt ist (z.B. durch Quarternierung), fügt man der wäßrigen Füllstoffsuspension die berechnete Menge an Säuren oder Quarternierungsmitten, bevorzugt Phosphorsäure, zur Aminsalzbildung zu oder nutzt im Falle der Verwendung von Braunkohle oder Torf als Füllstoffe die darin enthaltenen Huminsäuren zur Salzbildung unter Ausbildung von Polyurethan-Humaten.

Die so hergestellten, erfindungsgemäßen Trägermassen sedimentieren je nach Zusammensetzung verschieden schnell, aber vollständig in Wasser.

A)3. Allgemeine Vorschrift für die kontinuierliche Verfahrensweise (KV)

Als Apparatur dient ein Doppelpaddel-Schneckentrogmit einem Fassungsvolumen von ca. 180 l und einer Länge von ca. 300 cm, die mit einem Heizmantel für Warmwasser oder Dampf ausgerüstet ist und dessen Paddelwellen sich gegenläufig drehen. Die Produktförderung erfolgt zwangsweise von der Eintragsöffnung in Richtung Austragsöffnung, wobei zwischen den Paddelwellen eine gewisse Knetung bzw. Quetschung des Reaktionsgemisches erfolgt. Der zerkleinerte Polyurethanschaumstoff und gegebenenfalls die Füllstoffe und anderen Zusätze werden vorzugsweise getrennt über Dosierschnecken in den Schneckentrog gefördert. An gleicher Stelle wird mittels Kolbenpumpen das Wasser und mittels Zahnradumpen das NCO-Prepolymer eingetragen. Es ist zweckmäßig, die kationischen NCO-Prepolymere mit der etwa 10-fachen Menge Wasser von etwa 10 bis 25°C in einem Durchflußmischer oder statischen Mischer innerhalb weniger Sekunden intensiv zu vermischen, weil dadurch die Füllstoffe, insbesondere der vorgetrocknete Braunkohlenstaub oder Torf, außerordentlich schnell und gleichmäßig mit der getrennt zudosierten Restmenge des (gegebenenfalls bis 90°C erhitzten) Wassers benetzt wird und die NCO-Prepolymeren in feinstverteilter Form die Feststoffe und Schaumstoffe gleichmäßig umhüllen.

In den meisten Fällen genügt eine Verweilzeit im Schneckentrog von ca. 1—8 Minuten, vevorzugt wird eine solche von etwa 2 Minuten eingestellt. Das gegebenenfalls mit Katalysatoren und/oder erhöhter Temperatur hergestellte Material wird durch eine am Ende des Troges unten befindliche Öffnung in Behälter ausgetragen und direkt oder zu einem späteren Zeitpunkt in Wasser suspendiert oder zur vollständigen Quellung mit Wasser besprüht.

A)4. Vergleichsbeispiele (nicht erfindungsgemäß)

Es werden Reaktionen der NCO-prepolymeren (deren Zusammensetzung siehe Tabelle 1) in

14

Überschußmengen Wasser, jedoch ohne Verwendung von Schaumstoffen oder anderen Füllstoffen zu Polyurethanharnstoffen umgesetzt.

a) Verwendung hydrophiler NCO-Prepolymerer

Rührt man die NCO-Prepolymeren (KIPP-A, KIOPP-B, BIPP-C oder IPP-D) bei Raumtemperatur in die 5- bis 10-fache Gewichtsmenge Wasser ein (wie dies bei den erfindungsgemäßen Patentbeispielen auch erfolgt, jedoch dort unter Zusatz von Schaumstoffteilchen und gegebenenfalls anderen Füllstoffen), so erhält man zunächst eine wäßrige Lösung, aus der innerhalb von 1—2 Minuten in bekannter Weise ein PU-Schaum-Gel gebildet wird, das auch nach wochenlanger Lagerung im Wasser aufschwimmt, d.h. sich nicht absetzt und das somit nach der Fließ- oder Wirbelbett-Technik als Träger bei Biokonversionsprozessen nicht verwendbar ist. Bei 50°C und darüber erfolgt die Schaum-Gel-Bildung sogar schon innerhalb von 20 Sekunden, wobei der Anteil geschlossener Zellen noch größer ist, was eine Anwendbarkeit noch weiter verschlechtert.

b) Verwendung hydrophober, kationischer NCO-Prepolymerer

In der 5- bis 10-fachen Menge Wasser reagieren die kationischen, hydrophoben NCO-Prepolymeren wie KOPP1-E und KOPP2-F bei Raumtemperatur deutlich langsamer und lassen sich zunächst in Wasser feine dispergieren. Innerhalb von Stunden bildet sich im Reaktionsgefäß allerdings ein erfindungsgemäß nicht verwertbarer, pulverförmiger Niederschlag.

Bei Temperaturen bei 50°C und darüber erfolgt die Niederschlagsbildung schon innerhalb von 40 Sekunden.

c) Verwendung hydrophober, nichtionischer NCO-Prepolymerer

In großen Überschußmengen Wasser ist eine Dispersion er NCO-Prepolymeren nicht möglich, vielmehr tritt bereits bei Raumtemperatur sofort eine Verfestigung zu einer mehr oder weniger klebrigen Masse ein, die allmählich erstarrt.

Beispiel 1
(Ausführliches Herstellungsbeispiel)

Trägermasse

6,5 Gewichtsteile eines Weichschaumstoffgranulats WSB-90 und 9,44 Gewichtsteile einer nativen Braunkohle aus dem Aachener Braunkohlerevier mit 11% Restfeuchte, welche zu Teilchen unter 100 μm zerkleinert wurde und somit als Brankohlenstaub vorliegt, werden in 80 Gewichtsteilen Wasser von 18°C verrührt und nach dem allgemeinen Herstellungsverfahren A2, wie oben angegeben, mit 5,0 Gew.-Teilen des kationischen NCO-Prepolymeren KIPP-A (5,1 Gew.-% NCO (siehe Tabelle 1)) verrührt. Es entsteht ein Trägermaterial in Form eines wassergequollenen, sich feucht anfühlenden, leicht elastischen Feststoffes, der in Stücke unter einer Größe von 12 mm granuliert wird. Das feste, mit Schaumstoff und Braunkohle gefüllte kationische Polyurethan(harnstoff)-Trägermaterial enthält 42,5 g Braunkohle-Trockensubstanz und 32,5 g PU-Schaumstoff in 100 g wasserfrei angenommener Trägermasse (aus wasserfreie bereichneter Braunkohle, PU-Schaumstoff und wasserfrei angenommener Polyurethanharnstoffe), folglich 75 Gew.-% an Schaumstoff plus Braunkohle, bezogen auf Trokkenmasse des gefüllten Polyurethan(harnstoff)-Trägers.

Das erhaltene, granulierte Trägermaterial wird nun mit überschüssigem Wasser versetzt, 24 Stunden (bei Raumtempertur) vollständig gequollen und gegebenenfalls das darüber stehend Wasser abdekantiert. Der hieraus abgeleitete Wert, der die Gewichtsprozentmenge Wassers im und zwischen dem gequollenen Träger (füllstoffhaltigem Polyurethanharnstoff) angibt, wird hier als *Wasseraufnahmefähigkeit (WAF)* bezeichnet. (siehe nähere Erläuterung weiter unten).

Der Feststoffgehalt (TS(S)) der so hergestellten wäßrigen Suspension des Granulats in Form eines nunmehr stark gequollenen Trägermaterials beträgt im Beispielfall 100 g Feststoff pro Liter "Suspension" (ohne überstehendes Wasser). Der Feststoffgehalt in einem Liter einer der artigen Suspension (ohne überstehendes Wasser). Der Feststoffgehalt in einem Liter einer derartigen Suspension (ohne überstehendes Wasser) wird als *Trockensubstanz der Suspension* (abgekürzt *TS(S)*) bezeichnet.

Das Gewicht von einem Liter dieser Suspension des stark gequollenen Trägermaterials (ohne überstehendes Wasser) wird als *Suepensionsgewicht* (abgekürzt *SG*) bezeichnet.

Aus dem Gewicht von einem Liter der Suspension (SG) und dem Wert der darin enthaltenen Trockenmasse des Trägers (TS-S) wird der Wert des sogenannten *Suspensionsfaktors (F4)* abgeleitet. Der Wert des Suspensionsfaktors F4 minus 1 *(F4-1)* gibt an, die *wievielfache Menge Wasser* (bezogen auf Trägertrockensubstanz) in der Suspension insgesamt (als Quellwasser und als Wasser in den Zwischenräumen in oder zwischen den Trägerpartikeln) sich befindet.

Der Wert des Suspensionsfaktors F 4 wird in Praxis dadurch bestimmt, daß man die Trägertrockenmasse von einem Liter einer Suspension der Träger in Wasser (ohne überstehendes Wasser) bestimmt und das Gewicht der Suspension (SG) durch das Gewicht der darin enthaltenen Trägertrocken-masse (TS(S)) dividiert:

$$F4 = \frac{SG}{TS(S).}$$

15

Aus diesem Wert des Suspensionsfaktors F4 kann die Wasseraufnahmefähigkeit (WAF) als Charakteristikum für die erfindungsgemäß zu verwendenden Trägermassen nach folgender Formel bestimmt werden:

$$WAF = \frac{F4\ minus\ 1}{F4} \cdot 100;\ (in\ \%).$$

Dieser Wert der *Wasseraufnahmefähigkeit (WAF)* in Prozenten ausgedrückt, gibt ein anschauliches Bild für den Zustand der hochgequollenen und gegebenenfalls wasseraufnehmende Zwischenräume tragenden Trägermassen wieder, wie sie in dem gequollenen Zustand in der wäßrigen Suspension verwendet werden. Im Beispiel 1 beträgt beispielsweise der Trockensubstanzgehalt von 1 Liter der Suspension ohne überstehendes Wasser 108 g Feststoff. Bei einem Suspensionsgewicht von 1013 g pro Liter Suspension errechnet sich hieraus der Suspensionsfaktor

$$F4 = \frac{1004}{103} = 9.3.$$

Ein Gewichtsteil Trockensubstanz der Trägermasse wird somit mit der 8,3-fachen Wasermenge in die beschriebene gequollene Suspensionsform überführt. Anders ausgedrückt, berägt der Wert der Wasseraufnahmefähigkeit 8,3 durch 9,3 mal 100 $\doteq$ 89,2%.

Zur weiteren Charakterisierung der Trägermassen werden noch die Schüttgewichte (in g/l) nach unterschiedlichen Behandlungsarten bestimmt, wobei die Schüttgewichte S1 bis S3 unter folgenden Bedingungen ermittelt werden:

S1. Schüttgewicht, abgetropft:

Die Trägermasse wird 24 Stunden in einer großen Überschußmenge an Wasser supendiert, danach wird mit dieser gequollenen Masse ein Sieb mit 2 mm Sieblöchern 10 cm hoch gefüllt und 1 Stunde abtropfen gelassen; die verbleibende Füllmenge wird danach in einem Meßgefäß gewogen und auf das Schüttgewicht pro Liter umgerechnet.

S2. Schüttgewicht, ausgequetscht:

Die nach S1 abgetropfte Trägermasse wird auf einem 1 mm Sieb 5 Minuten lang einem Druck von 3 bar ausgesetzt und danach in einem Meßgefäß gewogen. Nach Umrechnung auf einen Liter wird das Schüttgewicht S2 ermittelt.

S3. Schüttgewicht, getrocknet:

Die feuchte, ausgequetschte Trägermasse wird (etwa) 1 Tag bei 100° unter Vakuum bis zur Gewichtskonstanz getrocknet und in einem Meßgefäß wie oben ausgewogen.

Im obven angeführten Beispiel betragen die so bestimmten Werte von S1 bis S3:

S 1 (abgetropft) 536 g/l

S 2 (ausgequetscht) 403 g/l

S 3 (getrocknet) 14 g/l.

Zur besseren Vergleichbarkeit der Werte werden noch folgende Faktoren definiert:

F1: Der *Volumenfaktor*, ist der Quotient aus dem Gewicht der in Wasser gequollenen, abgetropften Probe pro Liter (Schüttgewicht (S1)) und der ermittelten Gewichtsmenge Trockensubstanz einem Liter wäßriger Suspension (TS(S)).

$$F1 = \frac{S1}{TS(S)}$$

F2: Der *Quetschfaktor* ist entsprechend der Quotient der ausgequetschten Probe pro Liter (Schüttgewicht S2) und der Trockensubstanzmenge pro Liter Suspension.

$$F2 = \frac{S2}{TS(S)}.$$

F3: Der *Quellfaktor* ist der Quotient aus der Gewichtsmenge der abgetropften Probe (S1) und die nach vollständiger Entfernung des Wassers aus der *abgetropften* Probe ermittelten Gewichtsmenge der *Trockenmasse (TS(S1))*.

$$F3 = \frac{S1}{TS(S1)}$$

Die Volumen-, Quetsch- und Quelfaktoren sollen mindestens 2, bevorzugt mindestens 3, besonders bevorzugt mindestens 4 betragen. Die Obergrenzen der genannten Faktoren liegen etwa unterhalb 20, bevorzugt unter 15. Außerdem sollen sich die drei Faktoren der gleichen Probe möglichst wenig unterscheiden, d.h. maximal nur um das Dreifache, bevorzugt nur um das Doppelte unterschiedlich sein.

Die Ergebnisse von Beispiel 1 sind in Tabellen 2 und 3 tabellarisch miterfaßt.

1. Charakteristik der im Beispielteil als Füllstoffe verwendeten Schaumstoffe

a) Weichschaumstoffe

Bei den drei Weichschaumstoffen WSB 14, 90 und 61 wurden Mischungen verschiedener Raumgewichte (von ca. 15 bis ca. 110 kg/m³) aus der großtechnischen Polyether-Polyurethan-Block- und Formschaumstoff-Produktion verwendet.

WSB-14:

Das Trockenschüttgewicht des überwiegend aus Blockschaumstoff-Abfall bestehenden Weichschaums beträgt etwa 14 g/l. Teilchengröße von 1 mm bis 20 mm. Schüttgewichte nach Suspendierung in Wasser S1: 263 g/l S2: 101 g/l; S3: 14 g/l; TS-S (Gehalt an Trockensubstanz in Wassersuspension): 12,5 g/l; Suspension.

WSF-61:

Weichschaumsbfall aus Formenaustrieb, mit einem sehr hohen Anteil an Schwarte (zäher Außenhaut), Schüttgewichte S1: 365 g/l; S2: 199 g/l; S3: 61 g/l; Ts 51,7 g/l; Suspension.

WSB-90:

Blockweichschaumabfälle; Teilchengrößen 1 bis 12 mm; Schüttgewichte S1: 365 g/l; S2: 199 g/l; S3: 40 g/l; Trockensubstanz TS-S 35 g/l; Suspension.

b) Block-Hartschaumstoff-Abfälle

HSB-65

Die Schüttgewichte des aus Block-Hartschaumstoffabfällen bestehenden Granulats mit Teilchengrößen unter 2 mm betragen: S1: 896 g/l; S2: 457 g/l; S3: 65 g/l.

2. Vergleichsversuche mit unmodifizierten Schaumstoffen:

Nicht mit Polyurethanvorprodukten gebundene PU-Schaumstoffe sind für die Biokonversionsverfahren in der Praxis völlig unbrauchbar, da die Schaumstoffe obenauf schwimmen.

Auch bei einem weiteren Vergleichsversuch in einer Rührapparatur mit einem auf eine Größe unter 12 mm zerkleinerten, einheitlichen Blockweichschaum relativ hoher Dichte (Schüttgewicht 36 g/l; Raumgewicht 90 g/l) schwamm sogar noch nach drei Monaten Wasserlagerung bei kurzer Unterbrechung der Rührwirkung der größte Teil des Materials sofort obenauf. Die Anwendung eines Wirbelbettes war mit diesem Schaumstoff nicht möglich. PU-Hartschaumstoffe sind gleichfalls umgeeignet, da sie obenauf schwimmen und außerdem nicht spröde sind und zu Abrieb führen.

3. Herstellung des erfindungsgemäßen Trägermaterials (ausführliche Darstellung des Verfahrens A/2)

Die Herstellung der in den Patentbeispielen 1 bis 21 (außer Beispielen 2 und 8) verwendeten, hochgefüllten Polyurethan (harnstoff)-massen erfolgt etwa bei Raumtemperatur in diskontinuierlicher Weise entwerden in einem Intensivmischer, der aus einem zylindrischen Behälter besteht, der auf einem drehbaren Teller schräg befestigt ist undmit einem exzentrisch einbringbaren Rührwerk ausgestattet ist, das in engegengesetzter Richtung zur Tellerdrehung läuft, oder man verwendet horizontal montierte Mischer, die mit Pflugscharähnlichen Werkzeugen ausgerüstet sind.

Der zerkleinerte Polyurethanschaumstoff-Abfall und die erforderliche Wassermenge werden vorgelegt, dann der Füllstoff, z.B. Braunkohle, gleichmäßig eingemischt und danach in einem feinen Strahl das NCO-Prepolymer mit Hilfe einer Zahnradpumpe zudosiert. Gegebenenfalls erfolgt als Letztes die Zugabe des Katalysators K (in Form einer auf das Zwanzigfache verdünnten wäßrigen Emulsion). Das Rühren wird, nachdem alle Komponenten eingemischt sind, nach wenigen Minuten abgebrochen und das Trägermaterial in etwa 10 cm hoher Schicht 10 bis 90 Min. ausgebreitet, bis die Isocyanatreaktionen weitgehend beendet sind.

Das Trägermaterial wird mit viel Wasser mehrmals gewaschen und kann danach direkt oder zu einem späteren Zeitpunkt eingesetzt werden.

Die Zusammensetzung der Träger nach Beispielen 1 bis 24 sind in der Tabelle 2 wiedergegeben.

4. Eigenschaften der erfindungsgemäßen Trägermassen nach Beispielen 1 bis 24 siehe Tabelle 3.

Beispiele 2 bis 24 (s. Tabelle 2)

Hochgefüllte, langsam sedimentierende Polyurethan(harnstoff)massen aus Polyurethan-Weich- und Hart-Schaumstoffabfällen, weiteren Füllstoffen und NCO-Verbindungen

Ausführungsform siehe z.B Beispiel 1; Zusammensetzung der verwendeten NCO-Prepolymeren siehe Tabelle 1; Mengenverhältnisse bei der Umsetzung und Ergebnisse siehe Tabellen 2 und 3.

Tabelle 2 Zusammensetzung der hochgefüllten Polyurethan(Wasserstoff)-Trägermassen für die Patentbeispiele 1 bis 21
(diskontinuierliches Herstellungsverfahren nach A)2, außer in Beispielen 2 und 8, welche kontinuierlich nach A)3 hergestellt wurden).

| Patent-beispiele | Füllstoffe (Abfall)Schaumstoff Menge | Typ | Sieb-größe (mm) | Andere Füllstoffe Menge | Art | Sieb-größe (µm) | NCO-Prepolymer Menge | Art | %NCO-geh. | Wasser- Menge | Temp. | Zusatz Menge | Art | Trocken-substanz (g/l) Suspension (TS(S)) | Schüttgewichte abge-tropft S 1 | ausge-quetscht S 2 | getrock-net S 3 |
|---|---|---|---|---|---|---|---|---|---|---|---|---|---|---|---|---|---|
| 1 | 6,5 | WSB-90 | 12 | 8,5 | BKS | 100 | 5 | KIPP A | 5,1 | 80 | 18°C | - | - | 108 | 536 | 403 | 144 |
| 2 | 5,0 | WSB-90 | 12 | 20,0 | TDIR | 100 | 5 | KIOP B | 5,3 | 70 | 30°C | - | - | 143 | 636 | 500 | 246 |
| 3 | 10,0 | WSB-14 | 12 | 10,0 | BKK | 100 | 7,5 | KOPP2 F | 3,2 | 77,5 | 85°C | - | | 56 | 533 | 175 | 60 |
| 4 | 7,5 | WSB-14 | 20 | 10,0 | BKS | 100 | 3,0 | KIPP A | 5,1 | 78,5 | 18°C | 1 | K Fel | 62 | 593 | 283 | 69 |
| 5 | 7,5 | WSB-14 | 20 | 10,0 | BKS | 100 | 5,0 OPP I 77/72 2,5 KOPP1 E | | 5,9 | 75 | 35°C | | K | 56 | 366 | 213 | 58 |
| 6 | 7,5 | WSB-14 | 12 | 10,0 | SK | 90 | 7,5 | OPP I | | 75 | 35°C | | K | 59 | 368 | 164 | 60 |
| 7 | 7,5 | WSB-14 | 12 | 10,0 | BKS | 100 | 7,5 | OPP I | | 75 | 35°C | 1 | K KPUD | 54 | 382 | 180 | 56 |
| 7a* | 7,5 | WSB-14 | 12 | 10,0 | BKS | 100 | 7,5 | OPP I | | 75 | 35°C | 1 | K KPA | 56 | 354 | 187 | 58 |
| 8 | 10,0 | WSB-14 | 12 | 10,0 | T | 100 | 5,0 | KOPP1 E | 5,9 | 75 | 60°C | - | - | 68 | 460 | 184 | 55 |
| 9 | 5 | WSB-14 | 20 | 6 BKK 5 TDIR | | 100 | 3,5 KIPP A 1,5 IPP D | | 5,1 5,3 | 79 | | - | | 79 | 448 | 306 | 87 |
| 10 | 7,5 | WSB-14 | 12 | 10 | BK | 100 | 3,5 | KOPP1 E | 5,7 | 79 | | | K | 44 | 528 | 200 | 66 |
| 11 | 5 | WSB-14 | 20 | 5 BK 5 BKK | | 90 | 6 | KOPP2 F | 5,7 | 79 | | | K | 85 | 470 | 295 | 84 |
| 12 | 20 | WSP-61 | 12 | 10 | BK | 100 | 3,5 KIOPP B 1,5 OPP H | | 5,3 | 65 | | - | | 110 | 581 | 328 | 100 |

(Mengenangaben in Gewichtsteilen, bezogen auf Trockenmasse)
* = Vergleichsversuch

EP 0 151 937 B1

Fortsetzung

**Tabelle 2** Zusammensetzung der hochgefüllten Polyurethan(Wasserstoff)-Trägermassen für die Patentbeispiele 1 bis 24
(diskontinuierliches Herstellungsverfahren nach A)2, außer in Beispielen 2, 8, 22 und 23, welche kontinuierlich nach A)3 hergestellt wurden).

| Patent-beispiele | Füllstoffe | | | | | | NCO-Prepolymer | | | Wasser- | | Zusatz | | Trocken-substanz (g/l) Suspension (TS(S)) | Schüttgewichte | | |
| | (Abfall)Schaumstoff | | | Andere Füllstoffe | | | | | | | | | | | | | |
| | Menge | Typ | Sieb-größe (mm) | Menge | Art | Sieb-größe (µm) | Menge | Art | %NCO-geh. | Menge | Temp. | Menge | Art | | abge-tropft S 1 | ausge-quetscht S 2 | getrock-net S 3 |
|---|---|---|---|---|---|---|---|---|---|---|---|---|---|---|---|---|---|
| 13 | 7 | WSB-14 | 12 | 7 | AK | 30 | 5 | KIPP A | 5,1 | 80 | | – | | 60 | 471 | 201 | 66 |
| 14 | 7,5 | WSB-90 | 12 | – | | | 5 KIPP A / 2,5 IPP D | | 5,1 / 4,9 | 85 | | – | | 60 | 579 | 479 | 131 |
| 15 | 7,5 | WSB-14 | 12 | | | | 3,0 | KIPP A | 5,1 | 84,5 | | 5 | Fe-1 | 40 | 514 | 124 | 31 |
| 16 | 3,5 | HSB-65 | 2 | 25 | BK | 100 | 20 | KOPP2 F | 3,2 | 50 | | 1,5 | SIG | 112 | 581 | 328 | 151 |
| 17 | 10 | WSB-14 | 12 | 10 | BK | 100 | 4 KIPP A / 4 IPP D | | 5,1 / 4,9 | 72 | | – | | 58 | 610 | 428 | 86 |
| 18 | 7 | WSB-14 | 12 | 10 | BK | 100 | 5 | KIPP A | 5,1 | 76 | | 2 | Fe-2 | 74 | 631 | 346 | 90 |
| 19 | 10 | WSB-14 | 12 | 5 | BK | 100 | 5 | BIPP C | 5,6 | 78 | | 2 | SIW | 56,8 | 632 | 234 | 67 |
| 20 | 10 | WSB-14 | 6 | 10 | BK | 100 | 4 | KOPP1 E | 5,9 | 76 | | | K | 47 | 506 | 262 | 58 |
| 21 | 4,5 | WSB-14 | 12 | 11 | SK | 90 | 3,5 | KIPP A | 5,1 | 81 | | – | | 54 | 562 | 278 | 64 |
| 22 | 7,5 | WSB-14 | 25 | 15 | BK | 100 | 7,5 | KOPP 1 E | 5,9 | 70 | | – | – | 61 | 279 | 221 | 82 |
| 23 | 7,5 | WSB-14 | 12 | 15 | BK | 100 | 8,5 | KOPP 1 E | 5,9 | 70 | | – | – | 59 | 346 | 232 | 83 |
| 24 | 8 | WSB-14 | 12 | 11 | BK | 100 | 8 | (K)OPP I | 5,6 | 73 | | – | – | 63 | 462 | 280 | 82 |

(Mengenangaben in Gewichtsteilen, bezogen auf Trockenmasse)

Fortsetzung Tabellen 2 und 3
Mengenangaben in Gewichtsteilen, jeweils bezogen auf Feststoff

K    = 0,5 Gew.-% Dibutylzinndilaurat, bezogen auf NCO-Prepolymermenge
BK   = Braunkohle — nativ (in Gew.-Teilen *Trocken*substanz)
BKK  = Braunkohlekoks
T    = Schwarztorf (Restfeuchte 8,4 Gew.-% Wasser)
AK   = Aktivkohle
SK   = Steinkohlenstaub (Antrazitstaub)
KI   = Kationisch-hydrophiles NCO-Prepolymer
I    = Nichticaisches-hydrophiles NCO-Prepolymer
KO   = Kationisch-hydrophobes NCO-Prepolymer
(K)O = Kationenbildendes hydrophobes NCO-Prepolymer
TDIR = In Wasser ausgerührter TDI-Sumpfrückstand unter 200 µm gemahlen
Fe 1 = Ferromagnetisches Eisenoxid ($Fe_3O_4$), Teilchengröße ca. 1 µm
Fe 2 = Eisenoxidpigment ($Fe_2O_3$), Teilchengröße ca. 1 µm
SIG  = Kieselsol, 30 %ig in Wasser, spez. Oberfläche 200 $m^2$/g zugegeben
SIW  = Wasserglas, 10 %ige Lösung mit 5 Gew.-% Phosphorsäure, angeliert zugegeben
KPUD= Wäßrige, kationische Polyurethandispersion
KPA  = Wäßrige, kationische Polyamiddispersion.

Tabelle 3

Volumen-, Quetsch- und Quellfaktoren der hochgefüllten Polyurethan(harnstoff)-

Trägermassen (Beispiele 1 - 21)

| Beispiel | F 1 Volumen-Faktor | F 2 Quetsch-Faktor | F 3 Quell-Faktor | F 4 Suspensions-Faktor | % WAF Wasseraufnahme | % FKS Feststoffgehalt |
|---|---|---|---|---|---|---|
| 1 | 5,0 | 3,7 | 4,8 | 9,3 | 89,2 | 10,8 |
| 2 | 4,4 | 3,5 | 3,1 | 7,4 | 86,1 | 13,9 |
| 3 | 9,5 | 3,1 | 6,1 | 18,2 | 94,5 | 5,5 |
| 4 | 9,6 | 4,6 | 5,7 | 16,3 | 93,8 | 6,2 |
| 5 | 6,3 | 3,7 | 4,5 | 17,4 | 94,3 | 5,7 |
| 6 | 6,2 | 2,8 | 4,3 | 17,1 | 94,2 | 5,8 |
| 7 | 7,1 | 3,3 | 4,9 | 18,7 | 94,7 | 5,3 |
| 8 | 6,7 | 2,7 | 4,2 | 14,2 | 93,1 | 6,9 |
| 9 | 5,7 | 3,9 | 5,9 | 13,2 | 92,4 | 7,6 |
| 10 | 12,0 | 4,5 | 6,5 | 22,9 | 95,6 | 4,4 |
| 11 | 5,5 | 3,5 | 5,1 | 12,0 | 91,7 | 8,3 |
| 12 | 5,3 | 3,1 | 4,5 | 9,2 | 89,1 | 12,9 |
| 13 | 7,3 | 3,1 | 4,8 | 15,7 | 93,6 | 6,4 |
| 14 | 9,7 | 8,2 | 8,4 | 16,9 | 44,1 | 5,9 |
| 15 | 13,0 | 3,1 | 8,3 | 25,2 | 96,1 | 3,9 |

Tabelle 3   (Fortsetzung)

Volumen-, Quetsch- und Quellfaktoren der hochgefüllten Polyurethan(harnstoff)-
Trägermassen (Beispiele 1 - 21)

| Beispiel | F 1 Volumen-Faktor | F 2 Quetsch-Faktor | F 3 Quell-Faktor | F 4 Suspen-sions-Faktor | % WAF Wasseraufnahme | % FKS Feststoffgehalt |
|---|---|---|---|---|---|---|
| 16 | 3,6 | 3,4 | 3,1 | 7,0 | 88,9 | 11,1 |
| 17 | 10,5 | 7,4 | 5,6 | 17,4 | 94,3 | 5,7 |
| 18 | 8,5 | 4,7 | 6,3 | 13,7 | 92,7 | 7,3 |
| 19 | 11,1 | 4,1 | 6,5 | 17,7 | 94,4 | 5,6 |
| 20 | 10,7 | 6,1 | 6,1 | 21,3 | 95,3 | 4,7 |
| 21 | 10,4 | 5,1 | 6,1 | 18,7 | 94,7 | 5,3 |
| Mittelwert $\bar{X}$ = | 8,0 | 4,2 | 5,5 | 15,7 | 93,0 | 7,1 |
| Unter - Obergrenze | 3,6-13,0 | 2,7-8,2 | 3,1-4,8 | 7,0-25,2 | 86,1-96,1 | 3,9-13,9 |
| Nachtrag: | | | | | | |
| 22 | 4,6 | 3,6 | 3,3 | 16,4 | 93,4 | 6,1 |
| 23 | 5,9 | 3,9 | 3,5 | 16,9 | 94,1 | 5,9 |
| 24 | 9,6 | 4,0 | 2,9 | 16,3 | 93,1 | 6,3 |

EP 0 151 937 B1

Fortsetzung Tabelle 3

Die Differenz des % WAF-Wertes (Wasseraufnahmefähigkeit) auf 100 stellt den prozentualen Gehalt (% FKS) in der Suspension (ohne überstehendes Wasser) dar.

*Zu Beispiel 7a\** (mit kationischer Polymamiddispersion)

Es handelt sich um einen Vergleich zu Beispiel 7, in dem zusätzlich eine kationische Polyurethandispersion zugegeben wurde und um einen Vergleich zu Beispiel 5, in dem ein Drittel der NCO-Prepolymeren durch ein kationisches Prepolymer ersetzt ist.

Die in Beispiel 7a verwendete kationische Polyamiddispersion wird bedeutend stärker in der wäßrigen Suspension des Trägermaterials ausgewaschen als in Beispiel 7 mit einer kationischen Polyurethandispersion und ist deshalb weniger geeignet. Sehr gut ist der Träger von Beispiel 5. Ohne jegliche Kationenanwesenheit erfolgt innerhalb weniger Stunden eine Braunfärbung (Huminsäuren-Lösung) der wäßrigen Phase, die den Träger für die meisten Verwendungsmöglichkeiten praktisch unbrauchbar macht.

Trägermaterial Beispiel 22 und 23
Vorschrift für die kontinuierliche Herstellung
Zusammensetzung siehe Tabelle 2, S. 72

Als Apparatur dient ein Doppelpaddel-Schneckentrog mit einem Fassungsvolumen von ca. 180 l und einer Länge von ca. 300 cm, und dessen Paddelwellen sich gegenläufig drehen. Die Produktförderung erfolgt zwangsweise von de Eintragsöffnung in Richtung Austragsöffnung, wobei zwischen den Paddelwellen eine gewisse Knetung bzw. Quetschung des Reaktionsgemisches erfolgt. Der unter 12 bzw. 25 mm zerkleinerte Polyurethanschaumstoff und der Braunkohlenstaub werden getrennt über Dosierschnecken in den Schneckentrog gefördert. An gleicher Stelle wird mittels Kolbenpumpen das Wasser und mittels Zahnradpumpen das NCO-Prepolymer eingetragen. Es ist zweckmäßig, aber nicht unbedingt erforderlich, das kationische NCO-Prepolymere mit der etwa 10fachen Menge Wasser von etwa 10 bis 25°C in einem Durchflußmischer oder statischen Mischer innerhalb weniger Sekunden intensiv zu vermischen, weil dadurch der vorgetrocknete Braunkohlenstaub außerordentlich schnell und gleichmäßig mit der getrennt zudosierten Restmenge des auf 50°C erhitzten Wassers benetzt wird und die NCO-Prepolymeren in feinstverteilter Form die Feststoffe und Schaumstoffe gleichmäßig umhüllen.

Nach einer Verweilzeit im Schneckentrog von 2—3 Minuten wird durch eine am Ende des Troges unten befindlichen Öffnung in bis zur Hälfte mit Wasser gefüllten Behältern ausgetragen und nach etwa 2 Stunden oder später mit Wasser gewaschen.

**Patentansprüche**

1. Verfahren zur Herstellung von schaumstoffhaltigen Polyurethan(harnstoff)-Massen aus NCO-Prepolymeren und Wasser, dadurch gekennzeichnet, daß man

A) di- und/oder mehrfunktionelle NCO-Prepolymere, vorzugsweise mit einer Funktionalität $\geq 2,1$, und einem NCO-Gehalt von 2 bis 12 Gew.-% NCO auf der Basis von

a) zwei- und/oder mehrfunktionellen, höhermolekularen Verbindungen mit gegenüber Isocyanaten reaktiven H-Atomen mit Molekulargewichten von 400 bis 12 000 und mit einer Gesamtfunktionalität von $\geq 2,1$, vorzugsweise Polyhydroxylverbindungen,

b) 0 bis 5 Mol pro Mol a) niedermolekularen, zwei- und/oder mehrwertigen Verbindungen des Molekulargewichts 32 bis 399, vorzugsweise 62 bis 254, und gegenüber Isocyanaten reaktiven H-Atomen, vorzugsweise Di- und/oder Polyolen,

c) kationische Gruppen aufweisenden oder kationische Gruppen bildenden Polyurethan-Ausgangskomponenten auf Basis der höhermolekularen Komponente a) und/oder der niedermolekularen Komponente b) und/oder Zusätzen von kationische Gruppen tragenden oder kationische Gruppen bildenden Polymeren, vorzugsweise mit quaternären Ammoniumgruppen oder salzbildenden tertiären Aminogruppen, und gegebenenfalls zusätzlich Anionengruppen in höchstens dem Kationenäquivalent entsprechender Menge,

d) überschüssigen Mengen bezogen auf a) bis c) an Di- und/oder Polyisocyanaten, vorzugsweise aromatischen Diisocyanaten, zur Ausbildung von NCO-Prepolymeren mit NCO-Gehalten von 2 bis 12 Gew.-% NCO,

B) gegebenenfalls mit Zusätzen von weiteren, niedermolekularen Polyisocyanaten zum NCO-Prepolymer A) in Mengen bis zum halben Gewichtsanteil von A) und bis zur Ausbildung von (A+B)-Mischungen mit NCO-Gehalten $\leq 30$ Gew.-% NCO,

C) in überstöchiometrischen Mengen an Wasser, vorzugsweise einer mindestens 2-fachen bis etwa 60-fachen Gewichtsmenge, bezogen auf Feststoffgehalt von A + B, gegebenenfalls in Mischung mit Polyaminen, wobei diese vorzugsweise bis maximal 50 Äquivalent-% der NCO-Gruppen in A/B verwendet werden,

D) unter Zusatz von vorgefertigten, stückigen, zerkleinerten Schaumstoffen, insbesondere PU-Schaumstoffen, vorzugsweise mit einem Raumgewicht von 10 bis 110 kg/m$^3$ und Teilchengrößen von etwa 1 mm bis etwa 30 mm bei stückigem Schaumstoff oder bis 20 mm dicken Schaumstoff-Folien,

E) gegebenenfalls unter weiterem Zusatz von Braunkohle und/oder Torf, vorzugsweise Braunkohlenpulver und/oder feinteiligem Schwarztorf in Mengen von 1 bis 90 Gew.-%, vorzugsweise ≥20 Gew.-%, besonders bevorzugt ≥20 bis 70 Gew.-%, bezogen auf die füllstoffhaltigen Polyurethan-(harnstoff)-Massen,

F) gegebenenfalls in Gegenwart von weiteren anorganischen und/oder organischen Füllstoffen und üblichen Zusatzstoffen, Katalysatoren und Hilfsmitteln der Polyurethanchemie, wobei E) und/oder F) in einer Menge von mindestens 2 Gew.-% vorliegen,

G) und gegebenenfalls in Gegenwart von Biomassen unter Einbau von lebenden Zellen oder intakten Enzymen, zu füllstoffhaltigen, in wäßrigem Milieu abriebfesten, stark quellfähigen, nicht ausblutenden, schaumstoffhaltigen, vorzugsweise stückigen Polyurethan(harnstoff)-Massen mit einem Wert der Wasseraufnahmefähigkeit (WAF-Wert) von 30 bis 97 Gew.-%, bezogen auf wassersgequollene, füllstoffhaltige Polyurethan(harnstoff)-Suspension ohne überstehendes Wasser, und einem Gehalt an kationischen Gruppen von 10 bis 3000, vorzugsweise 30 bis 1500 Milliäquivalenten kationischer oder kationbildender Gruppen pro 1000 g wasser- und füllstofffreier Polyurethan(harnstoff)-Masse, umsetzt.

2. Verfahren nach Anspruch 1, dadurch gekennzeichnet, daß man mehrfunktionelle NCO-Prepolymere A) mit einer Funktionalität ≥2,1, auf der Basis von

a) mehrfunktionellen Polyetherpolyolen,

b) 0 bis 2 Mol an niedermolekularen Di- und/oder Polyolen mit Molekulargewichten von 6 bis 399,

c) quarternäre Ammoniumgruppen und/oder salzbildende tertiäre Aminogruppen aufweisenden, Polyurethan-bildenden Ausgangskomponenten a) und/oder b) und

d) überschüssigen Mengen an aromatischen Polyisocyanaten, einsetzt.

3. Verfahren nach Ansprüchen 1 und 2, dadurch gekennzeichnet, daß man als mehrfunktionelle Polyetherpolyole hydrophobe Polyoxypropylenpolyole mit Oxyethylengehalten ≤20% einsetzt.

4. Verfahren nach Ansprüchen 1 bis 3, dadurch gekennzeichnet, daß man als vorgefertigte, stückige Schaumstoffe D) PU-Schaumstoffe, insbesondere PU-Weichschaumstoff(abfäll)e mit Raumgewichten von 10 bis 110 kg/m³ und Teilchengrößen von 1 bis 30 mm in Mengen von mindestens 5 Gew.-% und bis zu 95 Gew.-%, bezogen auf füllstoffhaltige Polyurethan(harnstoff)-Masse, einsetzt.

5. Verfahren nach Ansprüchen 1 bis 4, dadurch gekennzeichnet, daß man zusätzlich zu den Schaumstoffen D) als weitere Füllstoffe E) Braunkohle und/oder Torf und gegebenenfalls weitere organische und/oder anorganische Füllstoffe F) einsetzt und innerhalb der Polyurethan(harnstoff)-Träger einen Gehalt an kationischen und/oder kationenbildenden Gruppen von 30 bis 1500 Milliäqivalenten pro kg wasser- und füllstofffreier Polyurethan(harnstoff)-Masse einbaut.

6. Verfahren nach Ansprüchen 1 bis 5, dadurch gekennzeichnet, daß man als Zusatzstoffe 3—85 Gew.-% an vorgefertigten Schaumstoffteilchen D) und 2—90 Gew.-% an Füllstoffen E) und/oder F) verwendet, wobei die Anteile an D), E) und/oder F) so ausgewählt werden, daß die Summe aus Schaumstoff und Füllstoffen (D, E plus F) 5 bis 95 Gew.-% beträgt und eine NCO-Prepolymermenge von 5 bis 95 Gew.-% eingesetzt wird.

7. Verfahren nach Ansprüchen 1 bis 6, dadurch gekennzeichnet, daß man zusätzlich zu den Schaumstoffen D) Braunkohlenstaub und/oder Schwarztorf als Füllstoffe E) einsetzt und einen Gehalt an kationischen oder kationenbildenden Gruppen von 50 bis 750 Milliäquivalenten pro kg wasser- und füllstofffreier Polyurethan(harnstoff)-Masse einbaut.

8. Verfahren nach Ansprüchen 1 bis 7, dadurch gekennzeichnet, daß man neben den Polyurethan-Schaumstoffen D) 20 bis 70 Gew.-% Braunkohlenstaub und/oder Schwarztorf als Füllstoffe E) einbaut, wobei gegebenenfalls weniger als die Hälfte der Füllstoffe E) durch Füllstoffe F), insbesondere Steinkohlenstaub, Aktivkohle und/oder anorganische Füllstoffe, ersetzt sein können.

9. Vorgefertigte Schaumstoffteilchen aus Braunkohle und/oder Torf und/oder gegebenenfalls weitere Füllstoffe enthaltende Polyurethan(harnstoff)-Massen mit hohen Wasseraufnahmefähigkeitswerten nach dem Verfahren der Ansprüche 1—8, dadurch gekennzeichnet, daß der Schaumstoff- und Braunkohle- und/oder Torf-Gehalt und/oder weiterer Füllstoffgehalt insgesamt 5 bis 95 Gew.-%, vorzugsweise 25 bis 90 Gew.-%, der Gehalt an kationische Gruppen oder kationische Gruppen bildenden Gruppen 30 bis 1500 Milliäquivalente pro 1000 g wasser- und füllstofffreier Polyurethan(harnstoff)-Massen und der Wasseraufnahmefähigkeitswert (WAF) 30 bis 97 Gew.-% Wasser, bezogen auf wassergequollene, füllstoffhaltige Polyurethan(harnstoff)-Suspension, beträgt.

10. Verwendung der vorgefertigten Schaumstoffteilchen aus Braunkohle und/oder Schwarztorf und/oder weitere Füllstoffe enthaltenden Polyurethan(harnstoff)-Massen nach den Ansprüchen 1 bis 9, als in Wasser gut suspendierbare, gegebenenfalls Biomassen eingebaut enthaltende Träger in Biokonversionsprozessen zur Herstellung organischer Verbindungen, als gegebenenfalls Pflanzenwuchsstoffe, Dünger oder Samen enthaltende Träger mit hohem Wasserbindevermögen für Pflanzenwachstum, als Filtriermittel für wäßrige Flüssigkeiten oder als Adsorbentien für nicht wasserlösliche Flüssigkeiten wie z.B. (Roh)Oel und Fette in Wasser.

**Revendications**

1. Procédé de production de compositions de polyuréthanne(urée) contenant une mousse, à partir de

24

NCO-prépolymères et d'eau, caractérisé en ce qu'on transforme

A) des NCO-prépolymères difonctionnels et/ou multifonctionnels, ayant de préférence une fonctionnalité égale ou supérieure à 2,1 et une teneur en NCO de 2 à 12% en poids sur la base

a) de composés difonctionnels et/ou multifonctionnels de poids moléculaire élevé, porteurs d'atomes d'hydrogène aptes à réagir vis-à-vis d'isocyanates, ayant des poids moléculaires de 400 à 12 000 et une fonctionnalité totale égale ou supérieure à 2,1, de préférence des composés polyhydroxyliques,

b) de 0 à 5 moles, par ole de a), de composés divalents et/ou polyvalents de bas poids moléculaires, compris dans la plage de 32 à 399, de préférence de 62 à 254, et porteurs d'atomes d'hydrogène aptes à réagir vis-à-vis d'isocyanates, de préférence des diols et/ou des polyols,

c) de composants de départ du type polyuréthanne porteurs de groupes cationiques ou formant des groupes cationiques, à base du composant a) de poids moléculaire élevé et/ou du composant b) de bas poids moléculaire et/ou d'additions de polymères portant des groupes cationiques ou formant des groupes cationiques, avec de préférence des groupes ammonium quaternaires ou des groupes amino tertiaires pouvant former un sel, et en outre, le cas échéant,

des groupes anioniques en une quantitée correspondant au maximum à l'équivalent cationique,

d) de quantités en excès, par rapport aux composants a) à c), de diisocyanates et/ou de polyisocyanates, de préférence de diisocyanates aromatiques, pour la formation de NCO-prépolymères ayant des teneurs en NCO de 2 à 12% en poids,

B) le cas échéant avec des additions d'autres polyisocyantes de bas poids moléculaire au NCO-prépolymère A) en quantités allant jusqu'à la moitié de la proportion en poid de A) et jusqu'à la formation de mélanges (A+B) ayant des teneurs en NCO inférieures ou égales à 30% en poids,

C) dans des quantité d'eau supérieures à la quantité stoechiométrique, de préférence dans une quantité en poids atteignant au moins un facteur 2 et s'élevant jusqu'à un facteur 60 environ, par rapport à la teneur en matières solides de A + B,

le cas échéant en mélange avec des polyamines, lesquelles sont utilisées de préférence jusqu'à un maximum de 50 équivalents % des groupes NCO dans A/B,

D) avec addition de mousses préformées divisées en morceaux, notamment de mousses de polyuréthanne, ayant de préférence une densité apparente de 10 à 110 kg/m$^3$ et des diamètres de particules d'environ 1 mm à environ 30 mm dans le cas de la mousse en morceaux ou jusqu'à 20 mm d'épaisseur dans le cas de feuilles de mousse,

E) le cas échéant avec, en outre, addition de lignite et/ou de tourbe, de préférence de lignite en poudre et/ou de tourbe noire finement divisée, en quantités de 1 à 90% en poids, de préférence en quantités égales ou supérieures à 20% en poids, notamment égales ou supérieures à 20—70% en poids par rapport aux compositions de polyuréthanne(urée) contenant une charge,

F) le cas échéant en présence d'autres charges inorganiques et/ou organiques et d'autres additifs, catalyseurs et substances auxiliaires d'emploi classique dans la chimie des polyuréthannes, les composants E) et/ou F) étant présents en une quantité d'au moins 2% en poids,

G) et, le cas échéant, en présence de biomasses avec incorporation de cellules vivantes ou d'enzymes intacts, en des compositions de polyuréthanne(urée) de préférence en morceaux, contenant une charge, résistant à l'usure par frottement en milieu aqueux, douées d'une grande aptitude au gonflement, sans phénomène de migration, contenant une mousse, avec une valeur de capacité d'absorption de l'eau (valeur CAE) de 30 à 97% en poids par rapport à la suspension de polyuréthanne(urée) gonflée dans l'eau, contenant une charge, en l'absence d'eau surnageante, et une teneur en groupes cationiques de 10 à 3000, de préférence de 30 à 1500 milli-équivalents de groupes cationiques ou cationogènes pour 1000 g de composition de polyuréthanne(urée) dépourvus d'eau et de charge.

2. Procédé suivant la revendication 1, caractérisé en ce qu'on utilise des NCO-prépolymères A) multifonctionnels ayant une fonctionnalité supérieure ou égale à 2,1, à base

a) de polyétherpolyols multifonctionnels,

b) de 0 à 2 moles % de diols et/ou de polyols de bas poids moléculaire, ayant des poids moléculaires de 62 à 399,

c) de composants de départ a) et/ou b) formant des polyuréthannes, porteurs de groupes ammonium quaternaires et/ou de groupes amino tertiaires formant un sel, et

d) de quantités en excès de polyisocyanates aromatiques.

3. Procédé suivant les revendications 1 et 2, caractérisé en ce qu'on utilise comme polyéther-polyols multifonctionnels des polyoxypropylène-polyols hydrophobes ayant des teneurs en oxyéthylène inférieures ou égales à 20%.

4. Procédé suivant les revendications 1 à 3, caractérisé en ce qu'on utilise comme mousses D) préfabriquées, en morceaux, des mousses de polyuréthanne, notamment des mousses (déchets) molles de polyuréthanne ayant des densités apparentes de 10 à 100 kg/m$^3$ et des diamètres de particules de 1 à 30 mm en quantités au moins égales à 5% en poids et allant jusqu'à 95% en poids par rapport à la composition de polyuréthanne(urée) contenant une charge.

5. Procédé suivant les revendications 1 à 4, caractérisé en ce qu'on utilise en plus des mousses D), comme autres charges E), du lignite et/o de la tourbe et, le cas échéant, d'autres charges F) organiques et/ou inorganiques et on incorpore dans les supports de polyuréthanne(urée) une teneur en groupes

25

cationiques et/ou cationogènes de 30 à 1500 milli-équivalents par kg de compositions de polyuréthanne(urée) sans eau et sans charge.

6. Procédé suivant les revendications 1 à 5, caractérisé en ce qu'on utilise comme additifs 3 à 85% en poids de particules D) de mousses préformées et 2 à 90% en poids de charges E) et/ou F), en choisissant les proportions de D), E) et/ou F) de manière que la somme de la mousse et des charges (D, E plus F) atteigne 5 à 95% en poids et que l'on utilise une quantité de NCO-prépolymère de 5 à 95% en poids.

7. Procédé suivant les revendications 1 à 6, caractérisé en ce qu'on utilise, en plus des mousses D), du lignite en poudre et/ou de la tourbe noire comme charges E) et on incorpore une teneur en groupes cationiques ou cationogènes de 50 à 750 milli-équivalents par kg de compositions de polyuréthanne(urée) sans eau et sans charge.

8. Procédé suivant les revendications 1 à 7, caractérisé en ce qu'on incorpore, à côté des mousses de polyuréthanne D), 20 à 70% en poids de lignite en poudre et/ou de tourbe noire comme charges E), et on peut alors remplacer, le cas échéant, moins de la moitié des charges E) par des charges F), notamment du lignite en poudre, du charbon actif et/ou des charges inorganiques.

9. Particules de mousse préformées à partir de compositions de polyuréthanne(urée) contenant du lignite et/ou de la tourbe et/ou, le case échéant, d'autres charges, ayant des valeurs élevées de capacité d'absorption d'eau, selon le procédé des revendications 1 à 8, caractérisées en ce que la teneur en mousse et/ou en lignite et/ou en tourbe et/ou la teneur en autre charge représente au total 5 à 95% en poids, de préférence 25 à 90% en poids, la teneur en groupes cationiques ou en groupes formant des groupes cationiques s'élève à 30—1500 milli-équivalents par 1000 g de compositions de polyuréthanne(urée) sans eau et sans charge et la valeur de capacité d'absorption d'eau (CAE) est de 30 à 97% en poids d'eau, par raport à la suspension de polyuréthanne(urée) gonflée à l'eau, contenant une charge.

10. Utilisation des particules de mousses préformées de compositions de polyuréthanne(urée) contenant du lignite et/ou de la tourbe noire et/ou d'autres charges selon les revendications 1 à 9, comme support pouvant être aisément mis en suspension dans l'eau, auquel sont éventuellement incorporées des biomasses, dans des processus de bioconversion pour la production de composés organiques, comme supports contenant éventuellement des substances de croissance des plantes, des engrais ou des graines, à haut pouvoir de fixation de l'eau, pour le développement de plantes, comme milieu filtrant pour des liquides aqueux et comme matières adsorbantes pour des liquides non hydrosolubles tels que, par exemple, des huiles (brutes) et des graisses dans l'eau.

**Claims**

1. A process for the production of foam-containing polyurethane (urea) masses of NCO prepolymers and water, characterized in that

A) di- and/or polyfunctional NCO prepolymers, preferably having a functionality of $\geq$ 2.1 and an NCO content of 2 to 12% by weight NCO, based on

a) di- and/or polyfunctional, relatively high molecular weight compounds containing isocyanate-reactive H atoms and having molecular weights of 400 to 12,000 and an overall functionality of $\geq$ 2.1, preferably polyhydroxyl compounds,

b) 0 to 5 mol per mol a) low molecular weight, difunctional and/or polyfunctional compounds having a molecular weight of 32 to 399 and preferably 62 to 254 and isocyanate-reactive hydrogen atoms, preferably di- and/or polyols,

c) polyurethane starting components containing cationic groups or forming cationic groups based on the relatively high molecular weight component a) and/or the low molecular weight component b) and/or additions of polymers bearing cationic groups or forming cationic groups, preferably containing quaternary ammonium groups or salt-forming tertiary amino groups, and optionally anionic groups in at most the quantity corresponding to the cationic equivalent,

d) excess quantities, based on a) to c), of di- and/or polyisocyanates, preferably aromatic diisocyanates, for forming NCO prepolymers having NCO contents of 2 to 12% by weight NCO,

B) optionally with additions of other, low molecular weight polyisocyanates to the NCO prepolymer A) in quantities of up to half the quantity by weight of A) and up to the formation of (A+B) mixtures having NCO contents of $\leq$30% by weight NCO, are reacted

C) in overstoichiometric quantities of water, preferably in at least 2 to about 60 times the quantity by weight, based on the solids content of A+B, optionally in admixture with polyamines which are preferably used in a quantity of up to at most 50 equivalent-% of the NCO groups in A/B,

D) with addition of preformed, piece-form, size-reduced foams, particularly PU foams, preferably having a density of 10 to 110 kg/m$^3$ and particle sizes of from about 1 mm to about 30 mm in the case of piece-form foam or up to 20 mm thick foam sheets,

E) optionally with further addition of lignite and/or peat, preferably lignite powder and/or finely divided black peat in quantities of 1 to 90% by weight, preferably in quantities of $\geq$20% by weight and more preferably in quantities of $\geq$20 to 70% by weight, based on the filler-containing polyurethane (urea) masses,

F) optionally in the presence of other inorganic and/or organic fillers and standard additives, catalysts and auxiliaries of polyurethane chemistry, E) and/or F) being present in a quantity of at least 2% by weight,

G) and optionally in the presence of biomasses with incorporation of living cells or intact enzymes, to form filler-containing, highly swellable, non-bleeding, foam-containing, preferably piece-form polyurethane (urea) masses which are abrasion-resistant in aqueous medium and have a water absorption value (WA value) of 30 to 97% by weight, based on water-swollen, filler-containing polyurethane (urea) suspension without supernatant water, and a content of cationic groups of 10 to 3,000 and preferably 30 to 1,500 milliequivalents cationic or cation-forming groups per 1000 g water- and filler-free polyurethane (urea) mass.

2. A process as claimed in claim 1, characterized in that polyfunctional NCO prepolymers A) having a functionality of $\geq$ 2.1 based on

a) polyfunctional polyether polyols,

b) 0 to 2 mol low molecular weight di- and/or polyols having molecular weights of 62 to 399,

c) polyurethane-forming starting components a) and/or b) containing quaternary ammonium groups and/or salt-forming tertiary amino groups and

d) excess quantities of aromatic polyisocyanates are used.

3. A process as claimed in claims 1 and 2, characterized in that hydrophobic polyoxypropylene polyols having oxyethylene contents of $\geq$ 20% are used as the polyfunctional polyols.

4. A process as claimed in claims 1 to 3, characterized in that PU foams, more particularly flexible PU foam (waste), having densities of 10 to 110 kg/m$^3$ and particle sizes of 1 to 30 mm are used as the preformed, piece-form foams D) in quantities of at least 5% by weight and up to 95% by weight, based on the filler-containing polyurethane (urea) mass.

5. A process as claimed in claims 1 to 4, characterized in that lignite and/or peat and optionally other organic and/or inorganic fillers F) are used as further fillers E) in addition to the foams D) and a content of cationic and/or cation-forming groups of 30 to 1,500 milliequivalents per kg water- and filler-free polyurethane (urea) mass is incorporated in the polyurethane (urea) support.

6. A process as claimed in claims 1 to 5, characterized in that 3 to 85% by weight preformed foam particles D) and 2 to 90% by weight fillers E) and/or F) are used as additives, the percentages by weight of D), E), and/or F) being selected so that the sum of foam and fillers (D, E plus F) is 5 to 95% by weight and a quantity of NCO prepolymer of 5 to 95% by weight is used.

7. A process as claimed in claims 1 to 6, characterised in that lignite dust and/or black peat are used as fillers E) in addition to the foams D) and a content of cationic or cation-forming groups of 50 to 750 milliequivalents per kg water- and filler-free polyurethane (urea) mass is incorporated.

8. A process as claimed in claims 1 to 7, characterized in that, in addition to the polyurethane foams D), 20 to 70% by weight lignite dust and/or black peat are incorporated as fillers E), less than half the fillers E) optionally being replaceable by fillers F), more particularly coal dust, active carbon and/or inorganic fillers.

9. Preformed foam particles of polyurethane (urea) masses containing lignite and/or peat and/or optionally other fillers and having high water absorption values obtained by the process claimed in claims 1 to 8, characterized in that the total content of foam and lignite and/or peat and/or other fillers is 5 to 95% by weight and preferably 25 to 90% by weight, the content of cationic groups or cation-forming groups is 30 to 1500 milliequivalents per 1,000 g water- and filler-free polyurethane (urea) masses and the water absorption value (WA value) is 30 to 97% by weight water, based on water-swollen, filler-containing polyurethane (urea) suspension.

10. The use of the preformed foam particles of polyurethane (urea) masses ocntaining lignite and/or peat and/or other fillers according to claims 1 to 9 as supports readily suspendable in water and optionally containing incorporated biomasses in bioconversion process for the production of organic compounds, as supports optionally containing plant growth promoters, fertilizers or seeds and having high water binding powder for plant growth, as a filtration medium for aqueous liquids or as adsorbents for water-insoluble liquids, such as for example (crude) oil and fats in water.